# EUROPEAN PATENT APPLICATION

(11) **EP 4 837 919 A1**
(43) Date of publication of application: **11.11.2026**
(21) Application number: 25736182.4
(22) Date of filing: 02.01.2025
(51) Int. Cl.: C07K 16/28, A61K 39/395, A61P 35/00

(54) **B7-H3 BINDING PROTEIN**

(30) Priority: 03.01.2024 CN 202410009195; 05.12.2024 CN 202411783315
(71) Applicant: Beijing Tide Pharmaceutical Co., Ltd., Beijing 100176 (CN)
(72) Inventor: LI, Wei, Beijing 100176 (CN); ZHANG, Min, Beijing 100176 (CN); WANG, Yanming, Beijing 100176 (CN); HE, Jingchang, Beijing 100176 (CN); ZHANG, Qi, Beijing 100176 (CN); GUO, Tingting, Beijing 100176 (CN)
(74) Representative: Novitas Patent AB
(86) International application number: PCT/CN2025/070139
(87) International publication number: WO 2025/146081

(57) **Abstract**

Provided is a B7-H3 binding protein, and in particular a nanobody and a heavy chain antibody that specifically bind to B7-H3, as well as a vector, a cell, or a nucleic acid that encodes the antibody, and a diagnostic or therapeutic use of said antibody.

## Description

The present International Patent Application claims priority to Chinese Patent Application No.202410009195.X filed on January 3, 2024, and Chinese Patent Application No. 202411783315.8 filed on December 5, 2024, the entire contents of which are incorporated herein by reference for all purposes.

### TECHNICAL FIELD

The present application belongs to the field of biotechnology, and in particular relates to antigen-binding proteins, heavy chain antibodies, and nanobodies, and uses thereof.

### BACKGROUND

B7-H3 (CD276) is a member of the B7 family of proteins and plays a key role in the development of cancer. As one of the targets of immune checkpoints, B7-H3 is selectively expressed on tumor cells and immune cells in the tumor microenvironment, and is involved in the proliferation and metastasis, and associated with therapeutic resistance of tumor cells.

The protein expression level of B7-H3 differs dramatically between normal tissues and tumor tissues. B7-H3 is highly expressed in various tumor tissues, such as prostate cancer, pancreatic cancer, hepatocellular carcinoma, etc., while it is rarely expressed in normal tissues (Zhou WT, Jin WL. B7-H3/CD276: An Emerging Cancer Immunotherapy. Front Immunol. 2021;12:701006.). Utilizing this property, targeting B7-H3 with drugs can specifically kill cancer tissues, while minimizing damage to healthy cells. These properties make B7-H3 a promising cancer therapeutic target.

Nanobody is a novel type of antibody, also known as a single domain antibody, which is obtained by cloning the variable domain of the heavy chain of heavy-chain antibody (VHH), which naturally lacks light chains, contained in animals such as Camelidae. Compared with a conventional monoclonal antibody, the nanobody has the advantages such as small molecular weight, good solubility, strong stability, low immunogenicity, strong penetrability, high specificity, simple humanization, high expression, and ease of production. Nanobodies have good application prospects in the fields of biotechnology and medicine, and nanobody drugs have currently been approved for marketing. The application of nanobody technology to develop therapeutic antibodies against B7-H3 shows a good prospect.

### SUMMARY OF THE INVENTION

The present disclosure provides a B7-H3 binding protein that specifically recognizes and binds to B7-H3, particularly a nanobody and a heavy chain antibody that specifically recognizes and binds to B7-H3 (also referred to as B7-H3 single domain antibody, or B7-H3 VHH antibody).

Thus, in a first aspect of the present disclosure, a B7-H3 binding protein is provided. The B7-H3 binding protein, according to an embodiment of the present disclosure, may specifically target and bind to B7-H3.

In some embodiments, the binding protein comprises an immunoglobulin single variable domain, wherein the immunoglobulin single variable domain comprises CDR1, CDR2, and CDR3 comprised in a VHH set forth in any one of SEQ ID NOs: 1-13. In some embodiments, the CDR1, CDR2, and CDR3 are defined according to Kabat, AbM, Chothia, or IMGT.

In some embodiments of the B7-H3 binding protein described above, the immunoglobulin single variable domain comprises CDR1, CDR2, and CDR3 defined according to AbM, wherein:
(1) the CDR1 comprises an amino acid sequence having at least 85%, at least 90%, at least 95%, or 100% identity to the amino acid sequence set forth in SEQ ID NO: 29, the CDR2 comprises an amino acid sequence having at least 85%, at least 90%, at least 95%, or 100% identity to the amino acid sequence set forth in SEQ ID NO: 30, and the CDR3 comprises an amino acid sequence having at least 85%, at least 90%, at least 95%, or 100% identity to the amino acid sequence set forth in SEQ ID NO: 31;
(2) the CDR1 comprises an amino acid sequence having at least 85%, at least 90%, at least 95%, or 100% identity to the amino acid sequence set forth in SEQ ID NO: 23, the CDR2 comprises an amino acid sequence having at least 85%, at least 90%, at least 95%, or 100% identity to the amino acid sequence set forth in SEQ ID NO: 24, and the CDR3 comprises an amino acid sequence having at least 85%, at least 90%, at least 95%, or 100% identity to the amino acid sequence set forth in SEQ ID NO: 25;
(3) the CDR1 comprises an amino acid sequence having at least 85%, at least 90%, at least 95%, or 100% identity to the amino acid sequence set forth in SEQ ID NO: 26, the CDR2 comprises an amino acid sequence having at least 85%, at least 90%, at least 95%, or 100% identity to the amino acid sequence set forth in SEQ ID NO: 27, and the CDR3 comprises an amino acid sequence having at least 85%, at least 90%, at least 95%, or 100% identity to the amino acid sequence set forth in SEQ ID NO: 28;
(4) the CDR1 comprises an amino acid sequence having at least 85%, at least 90%, at least 95%, or 100% identity to the amino acid sequence set forth in SEQ ID NO: 32, the CDR2 comprises an amino acid sequence having at least 85%, at least 90%, at least 95%, or 100% identity to the amino acid sequence set forth in SEQ ID NO: 33, and the CDR3 comprises an amino acid sequence having at least 85%, at least 90%, at least 95%, or 100% identity to the amino acid sequence set forth in SEQ ID NO: 34;
(5) the CDR1 comprises an amino acid sequence having at least 85%, at least 90%, at least 95%, or 100% identity to the amino acid sequence set forth in SEQ ID NO: 35, the CDR2 comprises an amino acid sequence having at least 85%, at least 90%, at least 95%, or 100% identity to the amino acid sequence set forth in SEQ ID NO: 36, and the CDR3 comprises an amino acid sequence having at least 85%, at least 90%, at least 95%, or 100% identity to the amino acid sequence set forth in SEQ ID NO: 37;
(6) the CDR1 comprises an amino acid sequence having at least 85%, at least 90%, at least 95%, or 100% identity to the amino acid sequence set forth in SEQ ID NO: 38, the CDR2 comprises an amino acid sequence having at least 85%, at least 90%, at least 95%, or 100% identity to the amino acid sequence set forth in SEQ ID NO: 39, and the CDR3 comprises an amino acid sequence having at least 85%, at least 90%, at least 95%, or 100% identity to the amino acid sequence set forth in SEQ ID NO: 40;
(7) the CDR1 comprises an amino acid sequence having at least 85%, at least 90%, at least 95%, or 100% identity to the amino acid sequence set forth in SEQ ID NO: 41, the CDR2 comprises an amino acid sequence having at least 85%, at least 90%, at least 95%, or 100% identity to the amino acid sequence set forth in SEQ ID NO: 42, and the CDR3 comprises an amino acid sequence having at least 85%, at least 90%, at least 95%, or 100% identity to the amino acid sequence set forth in SEQ ID NO: 43;
(8) the CDR1 comprises an amino acid sequence having at least 85%, at least 90%, at least 95%, or 100% identity to the amino acid sequence set forth in SEQ ID NO: 44, the CDR2 comprises an amino acid sequence having at least 85%, at least 90%, at least 95%, or 100% identity to the amino acid sequence set forth in SEQ ID NO: 45, and the CDR3 comprises an amino acid sequence having at least 85%, at least 90%, at least 95%, or 100% identity to the amino acid sequence set forth in SEQ ID NO: 46;
(9) the CDR1 comprises an amino acid sequence having at least 85%, at least 90%, at least 95%, or 100% identity to the amino acid sequence set forth in SEQ ID NO: 47, the CDR2 comprises an amino acid sequence having at least 85%, at least 90%, at least 95%, or 100% identity to the amino acid sequence set forth in SEQ ID NO: 48, and the CDR3 comprises an amino acid sequence having at least 85%, at least 90%, at least 95%, or 100% identity to the amino acid sequence set forth in SEQ ID NO: 49;
(10) the CDR1 comprises an amino acid sequence having at least 85%, at least 90%, at least 95%, or 100% identity to the amino acid sequence set forth in SEQ ID NO: 50, the CDR2 comprises an amino acid sequence having at least 85%, at least 90%, at least 95%, or 100% identity to the amino acid sequence set forth in SEQ ID NO: 51, and the CDR3 comprises an amino acid sequence having at least 85%, at least 90%, at least 95%, or 100% identity to the amino acid sequence set forth in SEQ ID NO: 52;
(11) the CDR1 comprises an amino acid sequence having at least 85%, at least 90%, at least 95%, or 100% identity to the amino acid sequence set forth in SEQ ID NO: 53, the CDR2 comprises an amino acid sequence having at least 85%, at least 90%, at least 95%, or 100% identity to the amino acid sequence set forth in SEQ ID NO: 54, and the CDR3 comprises an amino acid sequence having at least 85%, at least 90%, at least 95%, or 100% identity to the amino acid sequence set forth in SEQ ID NO: 55;
(12) the CDR1 comprises an amino acid sequence having at least 85%, at least 90%, at least 95%, or 100% identity to the amino acid sequence set forth in SEQ ID NO: 56, the CDR2 comprises an amino acid sequence having at least 85%, at least 90%, at least 95%, or 100% identity to the amino acid sequence set forth in SEQ ID NO: 57, and the CDR3 comprises an amino acid sequence having at least 85%, at least 90%, at least 95%, or 100% identity to the amino acid sequence set forth in SEQ ID NO: 58; or
(13) the CDR1 comprises an amino acid sequence having at least 85%, at least 90%, at least 95%, or 100% identity to the amino acid sequence set forth in SEQ ID NO: 59, the CDR2 comprises an amino acid sequence having at least 85%, at least 90%, at least 95%, or 100% identity to the amino acid sequence set forth in SEQ ID NO: 60, and the CDR3 comprises an amino acid sequence having at least 85%, at least 90%, at least 95%, or 100% identity to the amino acid sequence set forth in SEQ ID NO: 61.

In some embodiments, the immunoglobulin single variable domain comprises an amino acid sequence having at least 85%, at least 90%, at least 95%, or 100% identity to the amino acid sequence set forth in any one of SEQ ID NOs: 1-13, or consists of an amino acid sequence having at least 80%, at least 85%, at least 90%, at least 95%, or 100% identity to the amino acid sequence set forth in any one of SEQ ID NOs: 1-13.

In some embodiments of the present disclosure, the B7-H3 binding protein is monovalent, bivalent, or multivalent.

In some embodiments of the present disclosure, the B7-H3 binding protein is monospecific, bispecific, or multispecific.

In some embodiments of the present disclosure, the immunoglobulin single variable domain comprised in the B7-H3 binding protein comprises a heavy chain framework region, and at least a portion of the heavy chain framework region is derived from at least one of a mouse antibody, a human antibody, a primate antibody, and a mutant thereof. In some embodiments of the present disclosure, at least a portion of the heavy chain framework region comprised in the B7-H3 binding protein is derived from a human antibody. Preferably, the immunoglobulin single variable domain comprises an amino acid sequence having at least 80%, at least 85%, at least 90%, at least 95%, or 100% identity to the amino acid sequence set forth in any one of SEQ ID NOs: 14-20. More preferably, the immunoglobulin single variable domain comprises or consists of the amino acid sequence set forth in any one of SEQ ID NOs: 14-20.

In some embodiments of the present disclosure, the B7-H3 binding protein is a heavy chain antibody. In some preferred embodiments, the heavy chain antibody further comprises a human IgG Fc. In some more preferred embodiments, the heavy chain antibody further comprises a human IgG1 Fc. In some specific embodiments, the human IgG1 Fc comprises an amino acid sequence having at least 80%, at least 85%, at least 90%, at least 95%, or 100% identity to the amino acid sequence set forth in SEQ ID NO: 69. More preferably, the human IgG1 Fc comprises or consists of the amino acid sequence set forth in SEQ ID NO: 69. In some specific embodiments of the present disclosure, the heavy chain antibody comprises an amino acid sequence having at least 80%, at least 85%, at least 90%, at least 95%, or 100% identity to the amino acid sequence set forth in any one of SEQ ID NOs: 70-82 and SEQ ID NOs: 62-68. In some embodiments of the present disclosure, the heavy chain antibody comprises or consists of the amino acid sequence set forth in any one of SEQ ID NOs: 70-82 and SEQ ID NOs: 62-68.

In some embodiments of the present disclosure, the B7-H3 binding protein is a nanobody. In some embodiments, the nanobody comprises an amino acid sequence having at least 80%, at least 85%, at least 90%, at least 95%, or 100% identity to the amino acid sequence set forth in any one of SEQ ID NOs: 1-13. In some preferred embodiments, the nanobody comprises or consists of the amino acid sequence set forth in any one of SEQ ID NOs: 1-13. In some embodiments, the nanobody comprises an amino acid sequence having at least 80%, at least 85%, at least 90%, at least 95%, or 100% identity to the amino acid sequence set forth in any one of SEQ ID NOs: 14-20. In some preferred embodiments, the nanobody comprises or consists of the amino acid sequence set forth in any one of SEQ ID NOs: 14-20.

The nanobody having the amino acid sequence set forth in SEQ ID NO: 1 corresponds to clone C23 of the present disclosure; the nanobody having the amino acid sequence set forth in SEQ ID NO: 2 corresponds to clone A13 of the present disclosure; the nanobody having the amino acid sequence set forth in SEQ ID NO: 3 corresponds to clone A2 of the present disclosure; the nanobody having the amino acid sequence set forth in SEQ ID NO: 4 corresponds to clone A3 of the present disclosure; the nanobody having the amino acid sequence set forth in SEQ ID NO: 5 corresponds to clone A77 of the present disclosure; the nanobody having the amino acid sequence set forth in SEQ ID NO: 6 corresponds to clone A83 of the present disclosure; the nanobody having the amino acid sequence set forth in SEQ ID NO: 7 corresponds to clone B102 of the present disclosure; the nanobody having the amino acid sequence set forth in SEQ ID NO: 8 corresponds to clone B91 of the present disclosure; the nanobody having the amino acid sequence set forth in SEQ ID NO: 9 corresponds to clone C184 of the present disclosure; the nanobody having the amino acid sequence set forth in SEQ ID NO: 10 corresponds to clone C357 of the present disclosure; the nanobody having the amino acid sequence set forth in SEQ ID NO: 11 corresponds to clone C59 of the present disclosure; the nanobody having the amino acid sequence set forth in SEQ ID NO: 12 corresponds to clone D16 of the present disclosure; the nanobody having the amino acid sequence set forth in SEQ ID NO: 13 corresponds to clone A14 of the present disclosure; the nanobody having the amino acid sequence set forth in SEQ ID NO: 14 corresponds to clone VHH21 of the present disclosure; the nanobody having the amino acid sequence set forth in SEQ ID NO: 15 corresponds to clone VHH22 of the present disclosure; the nanobody having the amino acid sequence set forth in SEQ ID NO: 16 corresponds to clone VHH23 of the present disclosure; the nanobody having the amino acid sequence set forth in SEQ ID NO: 17 corresponds to clone VHH24 of the present disclosure; the nanobody having the amino acid sequence set forth in SEQ ID NO: 18 corresponds to clone VHH25 of the present disclosure; the nanobody having the amino acid sequence set forth in SEQ ID NO: 19 corresponds to clone VHH26 of the present disclosure; and the nanobody having the amino acid sequence set forth in SEQ ID NO: 20 corresponds to clone VHH27 of the present disclosure.

In a second aspect of the present disclosure, provided is a fusion protein comprising the B7-H3 binding protein according to the first aspect of the present disclosure.

In a third aspect of the present disclosure, provided is a nucleic acid molecule encoding the B7-H3 binding protein according to the first aspect of the present disclosure or the fusion protein according to the second aspect of the present disclosure. In some preferred embodiments, the nucleic acid molecule is DNA.

In a fourth aspect of the present disclosure, provided is an expression vector comprising the nucleic acid molecule according to the third aspect of the present disclosure. As described above, the nucleic acid molecule encodes the B7-H3 binding protein according to the first aspect of the present disclosure or the fusion protein according to the second aspect of the present disclosure. Therefore, an expression vector introduced into a host cell according to an embodiment of the present application may express the B7-H3 binding protein according to the first aspect of the present disclosure or the fusion protein according to the second aspect of the present disclosure under conditions suitable for protein expression. In some embodiments of the present disclosure, the expression vector is a prokaryotic expression vector or a eukaryotic expression vector.

In a fifth aspect of the present disclosure, provided is a cell comprising the nucleic acid molecule according to the third aspect of the present disclosure or the expression vector according to the fourth aspect of the present disclosure. In some embodiments of the present disclosure, the cell is obtained by introducing the expression vector according to the fourth aspect of the present disclosure into a host cell. In some embodiments of the present disclosure, the cell is a prokaryotic cell or a eukaryotic cell. In some embodiments of the present disclosure, the cell is a mammalian cell, such as a CHO cell.

In a sixth aspect of the present disclosure, a conjugate is provided. In some embodiments of the present disclosure, the conjugate comprises the B7-H3 binding protein according to the first aspect of the present disclosure or the fusion protein according to the second aspect of the present disclosure, and further comprises a therapeutic, diagnostic, or imaging agent conjugated to the B7-H3 binding protein or the fusion protein. In some embodiments, the B7-H3 binding protein of the first aspect of the present disclosure or the fusion protein of the second aspect of the present disclosure comprises a linker with the therapeutic, diagnostic, or imaging agent. In some embodiments, the B7-H3 binding protein according to the first aspect of the present disclosure or the fusion protein according to the second aspect of the present disclosure is conjugated to the therapeutic, diagnostic, or imaging agent via a linker. In some embodiments, the linker comprises a cleavable linker or a non-cleavable linker. In some embodiments, the linker is selected from MC (6-maleimidocaproyl), Val-Cit (valine-citrulline), PABC (p-amino-benzyloxycarbonyl), DMEA (dimethylethylamine), Val-Cit-PABC, MC-Val-Cit-PABC, MC-Val-Cit-PABC-DMEA, GGFG (glycine-glycine-phenylalanine-glycine), MC-GGFG, AcBut (4-(4-acetylphenoxy)-butyric acid), and AcBut-dimethylhydrazide, preferably MC-GGFG. In some embodiments of the present disclosure, the therapeutic agent is a small-molecule cytotoxic drug. In some embodiments, the therapeutic agent is selected from the group consisting of topoisomerase inhibitors, microtubule inhibitors, antibiotics, DNA synthesis inhibitors, RNA polymerase II inhibitors, and RNA spliceosome inhibitors, preferably topoisomerase inhibitors. In some embodiments, the therapeutic agent is selected from the group consisting of exatecan (DX8951), MMAE, MMAF, duocarmycin, DM1, DM4, SN-38, Dxd, calicheamicin, doxorubicin, and PBDs (benzodiazepines), preferably exatecan. In some embodiments, the moiety formed by the linker and the therapeutic agent has the following structure:

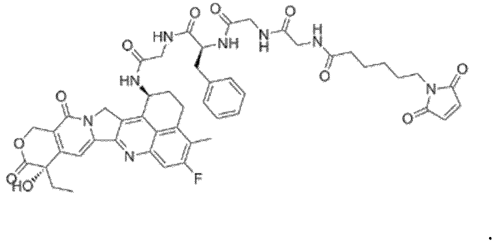

The conjugate according to the sixth aspect of the present disclosure may target and act on a target cell comprising B7-H3 under the guidance of the B7-H3 binding protein or the fusion protein.

In a seventh aspect of the present disclosure, provided is a composition comprising the B7-H3 binding protein according to the first aspect of the present disclosure, the fusion protein according to the second aspect of the present disclosure, the nucleic acid according to the third aspect of the present disclosure, the expression vector according to the fourth aspect of the present disclosure, the recombinant cell according to the fifth aspect of the present disclosure, and/or the conjugate according to the sixth aspect of the present disclosure. In some embodiments of the present disclosure, the composition is a pharmaceutical composition. In a preferred embodiment, the pharmaceutical composition further comprises a pharmaceutically acceptable carrier, excipient, or diluent.

In an eighth aspect of the present disclosure, provided is use of the B7-H3 binding protein according to the first aspect of the present disclosure, the fusion protein according to the second aspect of the present disclosure, the nucleic acid according to the third aspect of the present disclosure, the expression vector according to the fourth aspect of the present disclosure, the recombinant cell according to the fifth aspect of the present disclosure, the conjugate according to the sixth aspect of the present disclosure, and/or the composition according to the seventh aspect of the present disclosure in the manufacture of a medicament for preventing, treating, or alleviating a B7-H3-associated disease.

Also provided is use of the B7-H3 binding protein according to the first aspect of the present disclosure, the fusion protein according to the second aspect of the present disclosure, the nucleic acid according to the third aspect of the present disclosure, the expression vector according to the fourth aspect of the present disclosure, the recombinant cell according to the fifth aspect of the present disclosure, the conjugate according to the sixth aspect of the present disclosure, and/or the composition according to the seventh aspect of the present disclosure, in combination with another agent, in the manufacture of a medicament for preventing, treating, or alleviating a B7-H3-associated disease. In some embodiments, the another agent is an immunotherapeutic agent or a chemotherapeutic agent.

In a ninth aspect of the present disclosure, provided is the B7-H3 binding protein according to the first aspect of the present disclosure, the fusion protein according to the second aspect of the present disclosure, the nucleic acid according to the third aspect of the present disclosure, the expression vector according to the fourth aspect of the present disclosure, the recombinant cell according to the fifth aspect of the present disclosure, the conjugate according to the sixth aspect of the present disclosure, and/or the composition according to the seventh aspect of the present disclosure, for use in preventing, treating, or alleviating a B7-H3-associated disease.

In a tenth aspect of the present disclosure, provided is a method of preventing, treating, or alleviating a B7-H3-associated disease in a subject. In some embodiments, the method comprises administering to the subject the B7-H3 binding protein according to the first aspect of the present disclosure, the fusion protein according to the second aspect of the present disclosure, the nucleic acid according to the third aspect of the present disclosure, the expression vector according to the fourth aspect of the present disclosure, the recombinant cell according to the fifth aspect of the present disclosure, the conjugate according to the sixth aspect of the present disclosure, and/or the composition according to the seventh aspect of the present disclosure.

In some embodiments of the eighth to tenth aspects of the present disclosure, the B7-H3-associated disease or disorder is a B7-H3-mediated disease or disorder. In some embodiments, the B7-H3-mediated disease or disorder is a tumor. In some embodiments, the B7-H3-mediated disease or disorder is a solid tumor and/or a hematological tumor. In some specific embodiments, the B7-H3-mediated disease or disorder is one or more tumors selected from the group consisting of: lung cancer, breast cancer, prostate cancer, pancreatic cancer, colorectal cancer, melanoma, liver cancer, ovarian cancer, bladder cancer, gastric cancer, esophageal cancer, and kidney cancer. In some specific embodiments, the B7-H3 mediated disease or disorder is one or more tumors selected from the group consisting of adrenal tumor, AIDS-associated cancer, alveolar soft part sarcoma, astrocytic tumor, bone cancer, brain and spinal cord cancer, metastatic brain tumor, B-cell cancer, cancer, carotid body tumor, chondrosarcoma, chordoma, benign fibrous histiocytoma of the skin, desmoplastic small round cell tumor, ependymoma, Ewing's tumor, extraskeletal myxoid chondrosarcoma, osteogenesis imperfecta, osteofibrous dysplasia, gallbladder cancer or cholangiocarcinoma, gestational trophoblastic disease, germ cell tumor, head and neck cancer, hepatocellular carcinoma, islet cell tumor, Kaposi's sarcoma, leukemia, liposarcoma/malignant lipoma, lymphoma, medulloblastoma, meningioma, multiple endocrine neoplasia, multiple myeloma, myelodysplastic syndrome, neuroblastoma, papillary thyroid carcinoma, parathyroid adenoma, childhood cancer, peripheral nerve sheath tumor, melanocytoma, pituitary tumor, posterior uveal melanoma, renal metastatic cancer, rhabdoid tumor, rhabdomyosarcoma, sarcoma, skin cancer, soft tissue sarcoma, squamous cell cancer, gastric cancer, synovial sarcoma, testicular cancer, thymoma, and thyroid metastatic cancer. In a preferred embodiment, the B7-H3-mediated disease or disorder is a lung cancer.

In an eleventh aspect of the present disclosure, provided is a kit for detecting B7-H3 or a cell comprising B7-H3. In some embodiments, the kit comprises the B7-H3 binding protein according to the first aspect of the present disclosure, or the conjugate according to the sixth aspect of the present disclosure.

In a twelfth aspect of the present disclosure, provided is use of the B7-H3 binding protein according to the first aspect of the present disclosure or the conjugate according to the sixth aspect of the present disclosure in the manufacture of a kit for detecting B7-H3 or a cell comprising B7-H3.

In a thirteenth aspect of the present disclosure, provided is the B7-H3 binding protein according to the first aspect of the present disclosure, or the conjugate according to the sixth aspect of the present disclosure, for use in detecting B7-H3 or a cell comprising B7-H3.

In a fourteenth aspect of the present disclosure, provided is a method of detecting B7-H3 or a cell comprising B7-H3. In some embodiments of the present disclosure, the method comprises contacting the B7-H3 binding protein according to the first aspect of the present disclosure, or the conjugate according to the sixth aspect of the present disclosure, with a sample to be tested. Also provided is a method of determining the presence and/or amount of B7-H3, comprising contacting the B7-H3 binding protein according to the first aspect of the present disclosure, and/or the conjugate according to the sixth aspect of the present disclosure, with a sample to be tested.

In a fifteenth aspect of the present disclosure, provided is a chimeric antigen receptor. In some embodiments of the present disclosure, the chimeric antigen receptor comprises an antigen recognition domain comprising the B7-H3 binding protein according to the first aspect of the present disclosure, a hinge region, a transmembrane domain, and an intracellular domain comprising a costimulatory domain and a signaling domain.

Further aspects and advantages will be described below, at least part of which will become apparent from the following description taken in conjunction with the accompanying drawings, and/or will be apparent to those of ordinary skill in the art from the embodiments described hereinafter.

### BRIEF DESCRIPTION OF THE DRAWINGS

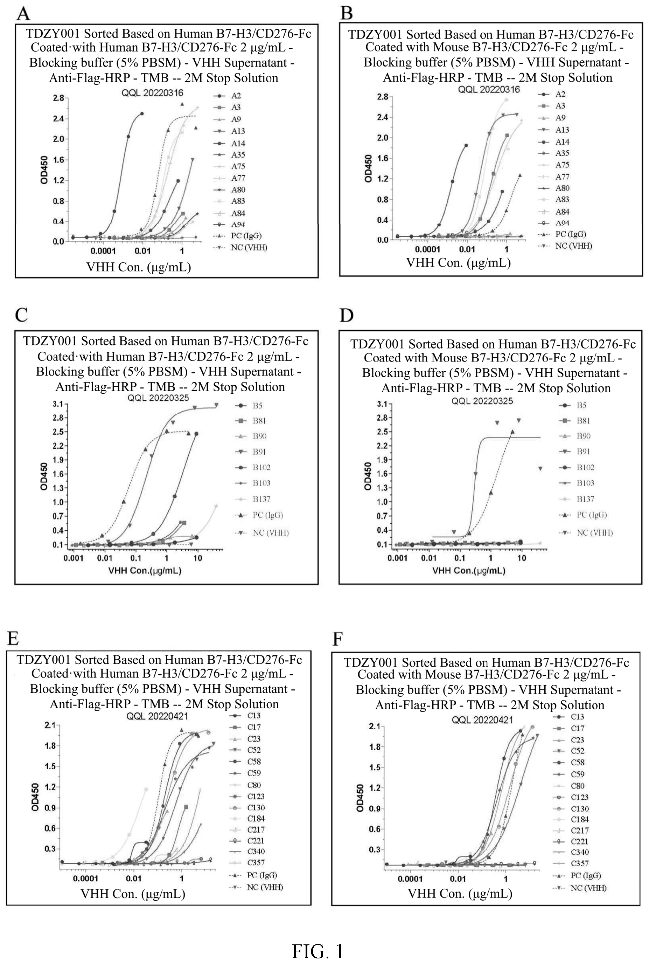

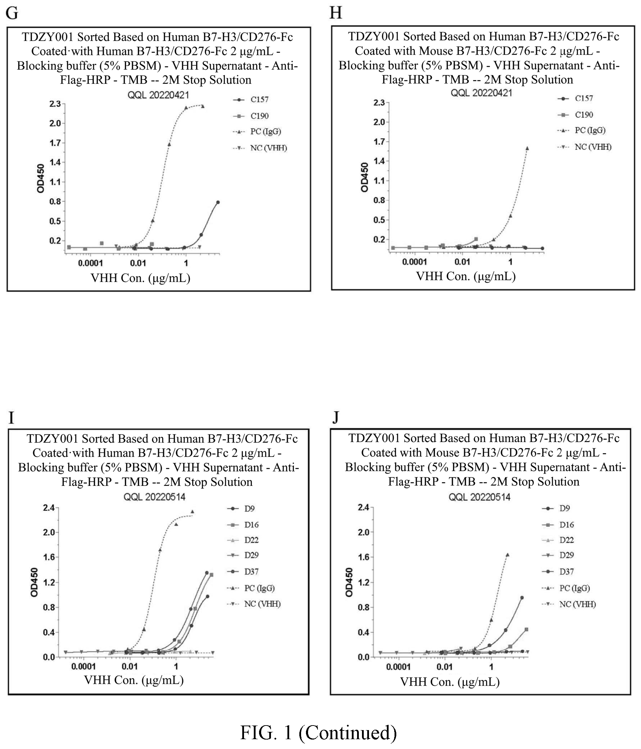

FIG. 1 shows the results of the ELISA assay of VHH supernatants expressed from 33 primarily screened clones with unique sequences using the corresponding Human B7-H3 antigen or Mouse B7-H3 antigen, respectively. FIG. 1A shows the results of the ELISA assay of VHH supernatants expressed from clones A2, A3, A9, A13, A14, A35, A75, A77, A80, A83, A84, and A94 using Human B7-H3-Fc antigen. FIG. 1B shows the results of the ELISA assay of VHH supernatants expressed from clones A2, A3, A9, A13, A14, A35, A75, A77, A80, A83, A84, and A94 using Mouse B7-H3-Fc antigen. FIG. 1C shows the results of the ELISA assay of VHH supernatants expressed from clones B5, B81, B90, B91, B102, B103, and B137 using Human B7-H3-Fc antigen. FIG. 1D shows the results of the ELISA assay of VHH supernatants expressed from clones B5, B81, B90, B91, B102, B103, and B137 using Mouse B7-H3-Fc antigen. FIG. 1E shows the results of the ELISA assay of VHH supernatants expressed from clones C13, C17, C23, C52, C58, C59, C80, C123, C130, C184, C217, C221, C340, and C357 using Human B7-H3-Fc antigen. FIG. 1F shows the results of the ELISA assay of VHH supernatants expressed from clones C13, C17, C23, C52, C58, C59, C80, C123, C130, C184, C217, C221, C340, and C357 using Mouse B7-H3-Fc antigen. FIG. 1G shows the results of the ELISA assay of VHH supernatants expressed from clones C157 and C190 using Human B7-H3-Fc antigen. FIG. 1H shows the results of the ELISA assay of VHH supernatants expressed from clones C157 and C190 using Mouse B7-H3-Fc antigen. FIG. 1I shows the results of the ELISA assay of VHH supernatants expressed from clones D9, D16, D22, D29, and D37 using Human B7-H3-Fc antigen. FIG. 1J shows the results of the ELISA assay of VHH supernatants expressed from clones D9, D16, D22, D29, and D37 using Mouse B7-H3-Fc antigen.
FIG. 2 shows the results of ELISA determination of VHH-Fc antibodies expressed and purified from constructs using Human B7-H3-His.
FIG. 3 shows the results of ELISA determination of VHH-Fc antibodies expressed and purified from constructs using Mouse B7-H3-Fc.
FIG. 4 shows the results of ELISA determination of VHH-Fc antibodies expressed and purified from constructs using Cyno B7-H3-His.
FIG. 5 shows the binding activity of humanized antibodies VHH21-Fc, VHH22-Fc, VHH23-Fc, VHH24-Fc, VHH25-Fc, VHH26-Fc, and VHH27-Fc to the antigen. FIG. 5A shows the binding activity of the humanized antibodies to Human B7-H3-His. FIG. 5B shows the binding activity of the humanized antibodies to Mouse B7-H3 His. FIG. 5C shows the binding activity of the humanized antibodies to Cyno B7-H3 His.
FIG. 6 shows the *in vivo* anti-tumor efficacy of the ADC molecule, humanized antibody VHH25-0143, in the Calu-6 human lung cancer subcutaneous transplanted tumor mouse model at different doses of 1 mg/kg, 3 mg/kg, and 10 mg/kg at a dosing frequency of once a week (QW) for a total of 3 doses.

### DETAILED DESCRIPTION

The aforementioned features and advantages of the present application, as well as additional features and advantages thereof, will be more clearly understood hereafter as a result of a detailed description of the following embodiments when taken in conjunction with the drawings.

The embodiments described herein with reference to drawings are explanatory, illustrative, and used to generally understand the present application. The embodiments shall not be construed to limit the scope of the present application. The same or similar elements and the elements having the same or similar functions are denoted by like reference numerals throughout the description.

Unless indicated or defined otherwise, all terms used have their usual meaning in the art, which will be clear to the skilled person. Reference is, for example, made to the standard handbooks.

Unless indicated otherwise, the term "immunoglobulin sequence", whether it is used herein to refer to a heavy chain antibody or a conventional 4-chain antibody, is used as a general term to include both the full-length antibody, single chains thereof, as well as all portions, domains or fragments thereof (including but not limited to antigen-binding domains or fragments such as V_{HH} domains or V_{H}/V_{L} domains, respectively). In addition, the term "sequence" as used herein (for example in terms like "immunoglobulin sequence," "antibody sequence," "variable domain sequence," "V_{HH} sequence," or "protein sequence"), should generally be understood as to include both the relevant amino acid sequence as well as the nucleic acid sequence or nucleotide sequence encoding the same, unless the context requires a more limited interpretation.

Unless indicated otherwise, all methods, steps, techniques, and manipulations that are not specifically described in detail can be performed and have been performed in a manner known per se, as will be clear to the skilled person. Reference is, for example, again made to the standard handbooks and the general background art mentioned herein, and to the further references cited therein.

For the purposes of comparing two or more nucleotide sequences, the percentage of "sequence identity" between a first sequence and a second sequence may be calculated by dividing [the number of nucleotides in the first sequence that are identical to the nucleotides at the corresponding positions in the second sequence] by [the total number of nucleotides in the first sequence] and multiplying by [100%], in which each deletion, insertion, substitution or addition of a nucleotide in the second nucleotide sequence - compared to the first nucleotide sequence - is considered as a difference at a single nucleotide (position).

Alternatively, the degree of sequence identity between two or more nucleotide sequences may be calculated using a known computer algorithm for sequence alignment, such as NCBI Blast v2.0, using standard settings.

Some other techniques, computer algorithms, and settings for determining the degree of sequence identity are described, for example, in WO 04/037999, EP 0 967 284, EP 1 085 089, WO 00/55318, WO 00/78972, WO 98/49185, and GB 2 357 768-A.

For the purposes of comparing two or more amino acid sequences, the percentage of "sequence identity" between a first amino acid sequence and a second amino acid sequence may be calculated by dividing [the number of amino acid residues in the first amino acid sequence that are identical to the amino acid residues at the corresponding positions in the second amino acid sequence] by [the total number of amino acids in the first amino acid sequence] and multiplying by [100%], in which each deletion, insertion, substitution or addition of an amino acid residue in the second amino acid sequence - compared to the first amino acid sequence - is considered as a difference at a single amino acid residue (position), i.e. as an "amino acid difference" as defined herein.

Alternatively, the degree of sequence identity between two amino acid sequences may be calculated using known computer algorithms, such as those mentioned above for determining the degree of sequence identity for nucleotide sequences, again using standard settings.

Usually, for the purpose of determining the percentage of "sequence identity" between two amino acid sequences in accordance with the calculation method outlined herein above, the amino acid sequence with the greatest number of amino acid residues will be taken as the "first" amino acid sequence, and the other amino acid sequence will be taken as the "second" amino acid sequence.

Also, in determining the degree of sequence identity between two amino acid sequences, the skilled person may take into account so-called "conservative" amino acid substitutions, which may generally be described as amino acid substitutions in which an amino acid residue is replaced with another amino acid residue of similar chemical structure and which has little or essentially no influence on the function, activity or other biological properties of the polypeptide. Such conservative amino acid substitutions are well known in the art, for example from WO 04/037999, GB-A-2 357 768, WO 98/49185, WO 00/46383 and WO 01/09300; and (preferably) types and/or combinations of such substitutions may be selected on the basis of the pertinent teachings from WO 04/037999 as well as WO 98/49185 and from the further references cited therein.

Such conservative substitutions are preferably substitutions in which one amino acid from the following groups (a) - (e) is substituted by another amino acid residue from the same group: (a) small aliphatic, nonpolar or slightly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, Ile, Val and Cys; and (e) aromatic residues: Phe, Tyr and Trp. Particularly preferred conservative substitutions are as follows: Ala to Gly or to Ser; Arg to Lys; Asn to Gln or to His; Asp to Glu; Cys to Ser; Gln to Asn; Glu to Asp; Gly to Ala or to Pro; His to Asn or to Gln; Ile to Leu or to Val; Leu to Ile or to Val; Lys to Arg, to Gln or to Glu; Met to Leu, to Tyr or to Ile; Phe to Met, to Leu or to Tyr; Ser to Thr; Thr to Ser; Trp to Tyr; Tyr to Trp; and/or Phe to Val, to Ile or to Leu.

Any amino acid substitutions applied to the polypeptides described herein may also be based on the analysis of the frequencies of amino acid variations between homologous proteins of different species developed by Schulz et al., Principles of Protein Structure, Springer-Verlag, 1978, on the analysis of structure-forming potentials developed by Chou and Fasman, Biochemistry 13: 211, 1974, and Adv. Enzymol., 47: 45-149, 1978, and on the analysis of hydrophobicity patterns in proteins developed by Eisenberg et al., Proc. Nat. Acad Sci. USA 81: 140-144, 1984; Kyte & Doolittle, J Mol. Biol. 157: 105-132, 1981, and Goldman et al., Ann. Rev. Biophys. Chem. 15: 321-353, 1986, all incorporated herein in their entirety by reference.

Information on the primary, secondary, and tertiary structure of nanobodies is given in the description herein and in the general background art cited above. Also, for this purpose, the crystal structure of a VHH domain from a llama is for example given by Desmyter et al., Nature Structural Biology, Vol. 3, 9, 803 (1996); Spinelli et al., Natural Structural Biology (1996); Vol. 3, 752-757; and Decanniere et al., Structure, Vol. 7, 4, 361 (1999). Further information is given on some of the amino acid residues that in conventional V_{H} domains form the V_{H}/V_{L} interface and potential camelization substitutions on these positions.

Amino acid sequences and nucleic acid sequences are designated as "identical" if they have 100% sequence identity (as defined herein) over their entire length.

A nucleic acid sequence or amino acid sequence is considered to be "(in) essentially isolated (form)" - for example, compared to its native biological source and/or the reaction medium or cultivation medium from which it has been obtained - when it has been separated from at least one other component with which it is usually associated in said source or medium, such as another nucleic acid, another protein/polypeptide, another biological component or macromolecule or at least one contaminant, impurity or minor component. In particular, a nucleic acid sequence or amino acid sequence is considered "essentially isolated" when it has been purified at least 2-fold, in particular at least 10-fold, more in particular at least 100-fold, and up to 1000-fold or more. A nucleic acid sequence or amino acid sequence that is "in essentially isolated form" is preferably essentially homogeneous, as determined using a suitable technique, such as a suitable chromatographical technique, such as polyacrylamide-gel electrophoresis.

The term "specificity" refers to the number of different types of antigens or antigenic determinants to which a particular antigen-binding molecule or antigen-binding protein (such as a nanobody or a polypeptide of the present disclosure) molecule bind. The specificity of an antigen-binding protein may be determined based on affinity and/or avidity. The affinity, represented by the equilibrium constant for the dissociation of an antigen with an antigen-binding protein (KD), is a measure for the binding strength between an antigenic determinant and an antigen-binding site on the antigen-binding protein: the lesser the value of the KD, the stronger the binding strength between an antigenic determinant and the antigen-binding molecule (alternatively, the affinity may also be expressed as the affinity constant (KA), which is 1/KD). Avidity is the measure of the strength of binding between an antigen-binding molecule (such as a nanobody, an antibody, or a heavy-chain antibody of the present disclosure) and the pertinent antigen. Avidity is related to both the affinity between an antigenic determinant and its antigen-binding site on the antigen-binding molecule and the number of pertinent binding sites present on the antigen-binding molecule. Typically, the antigen-binding protein (such as the nanobody and/or heavy-chain antibody of the present disclosure) will bind with a dissociation constant (KD) of 10⁻⁵ to 10⁻¹² moles/liter or less, and preferably 10⁻⁷ to 10⁻¹² moles/liter or less and more preferably 10⁻⁸ to 10⁻¹² moles/liter, and/or with a binding affinity of at least 10⁷ M⁻¹, preferably at least 10⁸ M⁻¹, more preferably at least 10⁹ M⁻¹, such as at least 10¹² M⁻¹. Any K_{D} value greater than 10⁻⁴ mol/liter is generally considered to indicate non-specific binding. Preferably, a B7-H3 antibody of the present disclosure, in particular a nanobody, will bind to the desired antigen with an affinity of less than 500 nM, preferably less than 200 nM, more preferably less than 10 nM, such as less than 500 pM. Specific binding of an antigen-binding protein to an antigen or antigenic determinant may be determined by any suitable means known *per se*, including, for example, Scatchard analysis and/or competitive binding assays, such as radioimmunoassays (RIA), enzyme immunoassays (EIA), and sandwich competition assays, and various variants thereof known *per se* in the art.

The term "immunoglobulin single variable domain" as used herein refers to an immunoglobulin variable domain that is capable of specifically binding to an epitope of an antigen without pairing with another immunoglobulin variable domain. One example of an immunoglobulin single variable domain of the present disclosure is a "domain antibody", such as the immunoglobulin single variable domain VH, and the immunoglobulin single variable domain VL (VH domain and VL domain). Another example of an immunoglobulin single variable domain is a camelid "VHH domain" (or simply "VHH") as defined below.

"Variable domain of Heavy chain of Heavy chain-only antibodies (i.e., of antibodies lacking light chains)" is also referred to as "VHH", also known as single domain antibodies, heavy chain single domain antibodies, VHH antibody fragments, and VHH antibodies. The term "VHH domain" is used to distinguish the variable domain from the heavy chain variable domain present in a conventional 4-chain antibody (referred to herein as a "VH domain") and the light chain variable domain present in a conventional 4-chain antibody (referred to herein as a "VL domain"). The VHH domain specifically binds an epitope without an additional antigen-binding domain (in contrast to the VH or VL domain in the conventional 4-chain antibody, where the epitope is recognized by the VL domain together with the VH domain). The VHH domain is a small, stable, and efficient antigen recognition unit formed by a single immunoglobulin domain.

In the context of the present disclosure, the terms "heavy chain single domain antibody," "VHH domain," "VHH," "VHH antibody fragment," "VHH antibody," and "nanobody" are used interchangeably.

As further described herein, the amino acid sequence and structure of a nanobody may be considered to, but not limited to, be comprised of four framework regions or "FRs", which are referred to in the art and herein as "Framework region 1" or "FR1"; as "Framework region 2" or"FR2"; as "Framework region 3" or "FR3"; and as "Framework region 4" or "FR4", respectively; these framework regions are interrupted by three complementarity determining regions or "CDRs", which are referred to in the art as "Complementarity Determining Region 1' or "CDR1"; as "Complementarity Determining Region 2" or "CDR2"; and as "Complementarity Determining Region 3" or "CDR3", respectively.

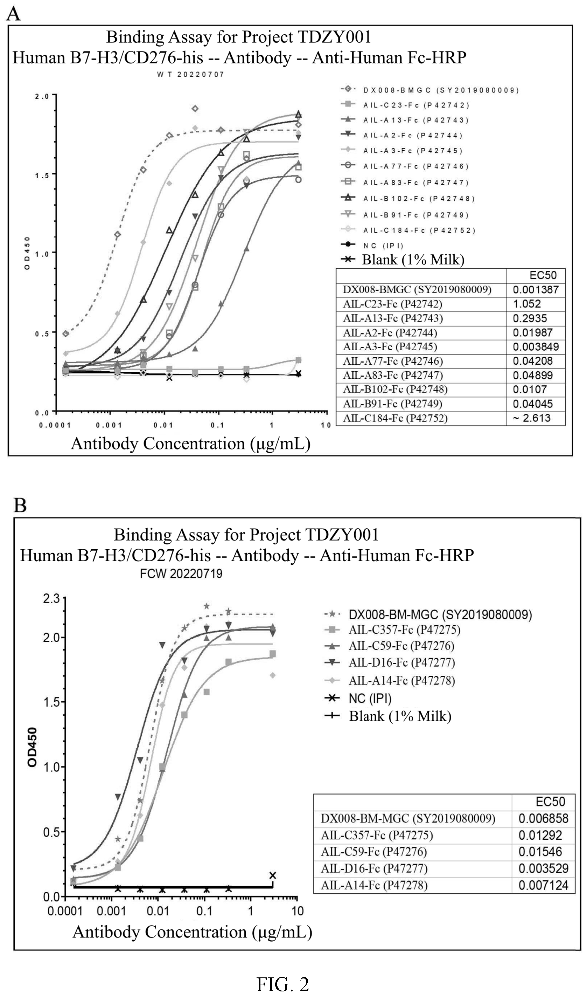

The amino acid residues of a nanobody are defined according to the general numbering for V_{H} domains given by Kabat et al. ("Sequence of proteins of immunological Interest", US Public Health Services, NIH Bethesda, MD, Publication No. 91), as applied to camelid V_{HH} domains in Riechmann and Muyldermans, J.Immunol.Methods 231,25-38(1999) (see, for example, FIG. 2 of said reference). In this respect, it should be noted that - as is well known in the art for V_{H} domains and for V_{HH} domains - the total number of amino acid residues in each of the CDRs may vary and may not correspond to the total number of amino acid residues indicated by the Kabat numbering. That is, one or more positions according to the Kabat numbering may not be occupied in the actual sequence, or the actual sequence may contain more amino acid residues than the number allowed for by the Kabat numbering. This means that, generally, the numbering according to Kabat may or may not correspond to the actual numbering of the amino acid residues in the actual sequence.

Alternative methods for numbering the amino acid residues of V_{H} domains, which may also be applied in a similar manner to V_{HH} domains from Camelids and to nanobodies, are the method described by Chothia et al. (Nature 342, 877-883 (1989)), the so-called "AbM definition," and the so-called "CONTACT definition". However, in the present specification, claims and drawings, the numbering according to IMGT of V_{HH} domains will be followed, unless indicated otherwise.

In accordance with the terminology used in the above references, the variable domains present in naturally occurring heavy chain antibodies will also be referred to as "V_{HH} domains", in order to distinguish them from the heavy chain variable domains present in conventional 4-chain antibodies (hereinafter referred to as "V_{H} domains") and from the light chain variable domains present in conventional 4-chain antibodies (hereinafter referred to as "V_{L} domains").

As mentioned in the prior art referred to above, V_{HH} domains have a number of unique structural characteristics and functional properties which make isolated V_{HH} domains (as well as nanobodies based thereon, which share these structural characteristics and functional properties with the naturally occurring V_{HH} domains) and proteins containing the same highly advantageous for use as functional antigen-binding domains or proteins. In particular, and without being limited thereto, V_{HH} domains (which have been "designed" by nature to functionally bind to an antigen in the absence of, and without any interaction with, a light chain variable domain) and nanobodies may function as a single, relatively small, functional antigen-binding structural unit, domain, or protein. This distinguishes the V_{HH} domains from the V_{H} and V_{L} domains of conventional 4-chain antibodies, which by themselves are generally not suited for practical application as single antigen-binding proteins or domains, but need to be combined in some form or another to provide a functional antigen-binding unit (as in, for example, conventional antibody fragments such as Fab fragments; in ScFv fragments, which consist of a V_{H} domain covalently linked to a V_{L} domain).

Because of these unique properties, the use of V_{HH} domains and nanobodies as single antigen-binding proteins or as antigen-binding domains (i.e. as part of a larger protein or polypeptide) offers a number of significant advantages over the use of conventional V_{H} and V_{L} domains, ScFvs or conventional antibody fragments (such as Fab- or F(ab')2-fragments): only a single domain is required to bind an antigen with high affinity and with high selectivity, so that there is no need for the presence of two separate domains present, nor to assure that these two domains are present in the right spatial conformation and configuration (i.e. through the use of specially designed linkers, as with ScFvs).

V_{HH} domains and nanobodies may be expressed from a single gene and require no post-translational folding or modification.

V_{HH} domains and nanobodies may be easily engineered into multivalent and multispecific formats.

V_{HH} domains and nanobodies are highly soluble and have no tendency to aggregate (as with "the mouse-derived antigen-binding domains" described by Ward et al., Nature, Vol. 341, 1989, p. 544).

V_{HH} domains and nanobodies are highly stable to heat, pH, proteases, and other denaturing agents or conditions (see, for example, Ewert et al, *supra*).

V_{HH} domains and nanobodies are easy and relatively cheap to prepare, even on a scale required for production. For example, V_{HH} domains, nanobodies, and proteins/polypeptides containing the same may be produced using microbial fermentation and do not require the use of mammalian expression systems, as with, for example, conventional antibody fragments.

V_{HH} domains and nanobodies are relatively small (approximately 15 kDa, or 10 times smaller than a conventional IgG) compared to conventional 4-chain antibodies and antigen-binding fragments thereof, and therefore show high(er) penetration into tissues (including but not limited to solid tumors and other dense tissues) than such conventional 4-chain antibodies and antigen-binding fragments thereof.

V_{HH} domains and nanobodies may show so-called cavity-binding properties (inter alia due to their extended CDR3 loop, compared to conventional V_{H} domains) and may therefore also access targets and epitopes not accessible to conventional 4-chain antibodies and antigen-binding fragments thereof.

As mentioned above, the present disclosure generally relates to nanobodies directed against B7-H3, as well as to polypeptides comprising one or more of such nanobodies (such as antibodies, heavy-chain antibodies, or antigen-binding fragments thereof), which are useful for the prophylactic, therapeutic, and/or diagnostic purposes described herein.

As also further described herein, the present disclosure further relates to nucleic acids encoding such nanobodies, B7-H3 binding proteins, or heavy chain antibodies, or antigen-binding fragments thereof, to methods for preparing such nanobodies, B7-H3 binding proteins, or heavy chain antibodies, or antigen-binding fragments thereof, to host cells expressing or capable of expressing such nanobodies, B7-H3 binding proteins, or heavy chain antibodies, or antigen-binding fragments thereof, to compositions comprising such nanobodies, B7-H3 binding proteins, or heavy chain antibodies, or antigen-binding fragments thereof, nucleic acids or host cells, and to uses of such nanobodies, B7-H3 binding proteins, or heavy chain antibodies, or antigen-binding fragments thereof, nucleic acids, host cells or compositions.

Generally, it should be noted that the terms nanobody, B7-H3 binding protein, or heavy chain antibody, or antigen-binding fragment thereof, as used herein have their broadest meaning and are not limited to a specific biological source or a specific method of preparation.

A humanized nanobody of the present disclosure may be as defined herein, but with the proviso that it has at least "one amino acid difference" (as defined herein) in at least one of the framework regions compared to the corresponding framework region of a naturally occurring V_{HH} domain. More specifically, according to a non-limiting aspect of the present disclosure, a nanobody may be as defined herein, but with the proviso that it has at least "one amino acid difference" (as defined herein) at least one of the Hallmark residues compared to the corresponding framework region of a naturally occurring V_{HH} domain. Usually, a nanobody will have at least one such amino acid difference with a naturally occurring VHH domain in at least one of FR2 and/or FR4, and in particular at least one of the Hallmark residues in FR2 and/or FR4.

A heavy chain antibody or HCAb consists of only two heavy chains, each comprising only the heavy chain variable region (VHH) along with a hinge region, CH2, and CH3. The antigen-antibody binding region of a heavy chain antibody or HCAb consists of three complementarity-determining regions, which give it better antigen binding ability than conventional antibodies. On the other hand, in addition to the lack of light chain, a major difference of the heavy chain antibody compared with ordinary antibodies is the absence of a CH1 region between its heavy chain variable region and the hinge region.

Another embodiment of the present application is a fusion protein comprising the B7-H3 binding protein described in the present disclosure. In some embodiments, in addition to the B7-H3 binding protein, the fusion protein further comprises one or more other biologically active proteins, which may be any protein of biological, therapeutic, prophylactic, or diagnostic significance or function that mediates a biological activity that prevents or alleviates a disease, condition, or disorder, when administered to a subject.

Another embodiment of the present disclosure is a nucleic acid capable of encoding a nanobody, B7-H3 binding protein, or heavy chain antibody as defined above.

Another embodiment of the present disclosure is an antibody-drug conjugate comprising a nanobody, or a B7-H3-binding protein or heavy chain antibody or antigen-binding fragment thereof, a linker, and a small molecule cytotoxic drug. Antibody-drug conjugate (ADC) is a chemical linkage that connects a bioactive small molecule drug to an antibody, e.g., the nanobody or the antibody of the present disclosure, which acts as a carrier to deliver the small molecule drug to target cells.

Another embodiment of the present disclosure is a composition comprising a nanobody, a B7-H3 binding protein, a heavy chain antibody, an antigen-binding fragment, a nucleic acid, a cell and/or an antibody-drug conjugate as defined above. Another embodiment of the present disclosure is a composition as defined above, further comprising a pharmaceutically acceptable carrier.

Another embodiment of the present disclosure is a B7-H3 binding protein, a heavy chain antibody, an antigen-binding fragment, or a nanobody as defined above, or a nucleic acid as defined above, or a cell as defined above, or an antibody-drug conjugate as defined above, or a composition as defined above, for use as a medicament.

Another embodiment of the present disclosure is a B7-H3 binding protein, a heavy chain antibody, an antigen-binding fragment or a nanobody as defined above, or a nucleic acid as defined above, or a cell as defined above, or an antibody-drug conjugate as defined above, or a composition as defined above, for use in treating, preventing, and/or alleviating a B7-H3-associated disease.

Another embodiment of the present disclosure is the use of a B7-H3 binding protein, a heavy chain antibody, an antigen-binding fragment or a nanobody as defined above, or a nucleic acid as defined above, or a cell as defined above, or an antibody-drug conjugate as defined above, or a composition as defined above, in the manufacture of a medicament for treating, preventing, and/or alleviating a B7-H3-associated disease.

In some embodiments, the B7-H3-associated disease or disorder is a B7-H3-mediated disease or disorder. In some embodiments, the B7-H3-mediated disease or disorder is a tumor. In some embodiments, the B7-H3-mediated disease or disorder is a solid tumor and/or a hematological tumor. In some specific embodiments, the B7-H3 mediated disease or disorder is one or more tumors selected from the group consisting of: lung cancer, breast cancer, prostate cancer, pancreatic cancer, colorectal cancer, melanoma, liver cancer, ovarian cancer, bladder cancer, gastric cancer, esophageal and renal cancer, adrenal tumor, AIDS-associated cancer, alveolar soft part sarcoma, astrocytic tumor, bone cancer, brain and spinal cord cancer, metastatic brain tumor, B-cell cancer, cancer, carotid body tumor, chondrosarcoma, chordoma, benign fibrous histiocytoma of the skin, desmoplastic small round cell tumor, ependymoma, Ewing's tumor, extraskeletal myxoid chondrosarcoma, osteogenesis imperfecta, osteofibrous dysplasia, gallbladder cancer or cholangiocarcinoma, gestational trophoblastic disease, germ cell tumor, head and neck cancer, hepatocellular carcinoma, islet cell tumor, Kaposi's sarcoma, leukemia, liposarcoma/malignant lipoma, lymphoma, medulloblastoma, meningioma, multiple endocrine neoplasia, multiple myeloma, myelodysplastic syndrome, neuroblastoma, papillary thyroid carcinoma, parathyroid adenoma, childhood cancer, peripheral nerve sheath tumor, melanocytoma, pituitary tumor, posterior uveal melanoma, renal metastatic cancer, rhabdoid tumor, rhabdomyosarcoma, sarcoma, skin cancer, soft tissue sarcoma, squamous cell cancer, gastric cancer, synovial sarcoma, testicular cancer, thymoma, and thyroid metastatic cancer. The cancer that may be treated by the B7-H3 binding protein, or the nanobody, or the heavy chain antibody of the present disclosure is not limited to the specific cancers described above, and all cancers expressing B7-H3 are also suitable for treatment with the B7-H3 binding protein, the nanobody, or the heavy chain antibody of the present disclosure.

Another embodiment of the present disclosure is use of the nanobody, the B7-H3 binding protein, the heavy chain antibody, the nucleic acid, the recombinant cell, the conjugate, or the composition as defined above, wherein said medicament is administered intravenously, subcutaneously, orally, sublingually, nasally, or by inhalation.

Another embodiment of the present disclosure is a method of prophylactically or therapeutically treating a B7-H39-associated disease or disorder, comprising administering to the patient an effective dosage of the nanobody, the antibody, the heavy-chain antibody, the fusion protein, the nucleic acid, the recombinant cell, the conjugate, or the composition as defined above.

Another embodiment of the present disclosure is a method of producing the B7-H3 binding protein, or the nanobody, or the heavy-chain antibody as defined above, comprising:
a) culturing host cells comprising nucleic acids capable of encoding the polypeptide as defined above under conditions allowing the expression of the polypeptide, and
b) recovering the produced polypeptide from the culture.

Another embodiment of the present disclosure is the method as defined above, wherein said host cells are bacterial, yeast, or mammalian cells.

Another embodiment of the present disclosure is a method of diagnosing a disease or disorder mediated by B7-H3, comprising the steps of:
a) contacting a sample with the B7-H3 binding protein, the nanobody, the heavy-chain antibody, the fusion protein, or the conjugate as defined above, and
b) detecting binding of said B7-H3 binding protein, nanobody, heavy-chain antibody, fusion protein, or conjugate to said sample, and
c) wherein a binding result above the cutoff value is indicative of a B7-H3-mediated disease or disorder in the sample.

In some embodiments, the sample is a biopsy sample. In some embodiments, the disease is a tumor. In some embodiments, the disease is a solid tumor and/or a hematoma. In some specific embodiments, the disease is one or more tumors selected from the group consisting of: lung cancer, breast cancer, prostate cancer, pancreatic cancer, colorectal cancer, melanoma, liver cancer, ovarian cancer, bladder cancer, gastric cancer, esophageal and renal cancer, adrenal tumor, AIDS-associated cancer, alveolar soft part sarcoma, astrocytic tumor, bone cancer, brain and spinal cord cancer, metastatic brain tumor, B-cell cancer, cancer, carotid body tumor, chondrosarcoma, chordoma, benign fibrous histiocytoma of the skin, desmoplastic small round cell tumor, ependymoma, Ewing's tumor, extraskeletal myxoid chondrosarcoma, osteogenesis imperfecta, osteofibrous dysplasia, gallbladder cancer or cholangiocarcinoma, gestational trophoblastic disease, germ cell tumor, head and neck cancer, hepatocellular carcinoma, islet cell tumor, Kaposi's sarcoma, leukemia, liposarcoma/malignant lipoma, lymphoma, medulloblastoma, meningioma, multiple endocrine neoplasia, multiple myeloma, myelodysplastic syndrome, neuroblastoma, papillary thyroid carcinoma, parathyroid adenoma, childhood cancer, peripheral nerve sheath tumor, melanocytoma, pituitary tumor, posterior uveal melanoma, renal metastatic cancer, rhabdoid tumor, rhabdomyosarcoma, sarcoma, skin cancer, soft tissue sarcoma, squamous cell cancer, gastric cancer, synovial sarcoma, testicular cancer, thymoma, and thyroid metastatic cancer.

Another embodiment of the present disclosure is a kit for diagnosing a B7-H3-associated disease or disorder. In some embodiments, the kit is useful in a method as defined above.

Another embodiment of the present disclosure is a B7-H3 binding protein, a nanobody, a heavy chain antibody, or a fusion protein as defined above, further comprising one or more *in vivo* imaging agents.

One embodiment of the present disclosure relates to a pharmaceutical composition comprising at least one B7-H3 binding protein, nanobody, heavy chain antibody, or fusion protein of the present disclosure and at least a pharmaceutically acceptable carrier, diluent, or excipient.

An ELISA assay to measure the binding of a B7-H3 binding protein, nanobody, heavy chain antibody, or fusion protein against B7-H3 to B7-H3 is well known.

One aspect of the present disclosure is an anti-B7-H3 polypeptide comprising at least one anti-B7-H3 heavy chain antibody, and in particular a nanobody derived therefrom. An aspect of the present disclosure is that such a polypeptide may comprise additional components. Such additional components may be polypeptide sequences, for example, one or more anti-B7-H3 nanobodies, or one or more anti-serum albumin nanobodies. Other fusion proteins are also within the scope of the present disclosure, and may include, for example, fusions to carrier polypeptides, signal molecules, tags, and enzymes. Other components may include, for example, radiolabels, organic dyes, or fluorescent compounds.

According to an aspect of the present disclosure, an anti-B7-H3 polypeptide of the present disclosure may comprise at least two identical or non-identical anti-B7-H3 nanobody sequences. The anti-B7-H3 polypeptide may comprise at least two of the aforementioned sequences with different affinities for B7-H3, thereby forming an anti-B7-H3 polypeptide combining a low-affinity binding sequence and a high-affinity binding sequence.

Methods of constructing bivalent polypeptides are known in the art (e.g. US 2003/0088074), and are also described below.

It may be desirable to modify the B7-H3 binding protein of the present disclosure with respect to effector function so as to enhance its therapeutic efficacy. For example, nanobody-fusions with certain Fc domains may be advantageous, especially with Fc domains of human origin.

In sequential administration, the polypeptide may be administered once or any number of times before and/or after administration of the agent, and in various doses. Sequential administration may be combined with simultaneous or sequential administration.

Another embodiment of the present disclosure is a B7-H3 binding protein as described herein, comprising one or more immunoglobulin single variable domains, wherein one or more of the immunoglobulin single variable domains are humanized.

By humanized is meant mutated so that potential immunogenicity upon administration in human patients is minor or nonexistent. Humanizing a polypeptide, according to the present disclosure, may comprise a step of replacing one or more of the non-human immunoglobulin amino acids by their human counterparts as found in a human consensus sequence or human germline gene sequence, without that polypeptide losing its typical character, i.e., the humanization does not significantly affect the antigen binding capacity of the resulting polypeptide.

According to one aspect of the present disclosure, a humanized nanobody is defined as a nanobody having at least 50% homology (e.g. 55%, 60%, 65%, 70%, 75%, 80%, 85%, 90%,95%, 98%, 100%) to a human framework region.

One embodiment of the present disclosure relates to a polypeptide comprising at least one nanobody wherein one or more amino acid residues have been substituted without substantially altering the antigen binding capacity.

The skilled person will recognize that the B7-H3 binding protein of the present disclosure may be modified, and such modifications are within the scope of the present disclosure. For example, the polypeptide may be used as a drug carrier, in which case it may be fused to a therapeutically active agent, or its solubility properties may be altered by fusion to ionic/bipolar groups, or it may be used in imaging by fusion to an appropriate imaging marker, or it may comprise modified amino acids, etc. The polypeptide may also be prepared as salts. Such modifications, which substantially retain the binding to B7-H3, are within the scope of the present disclosure.

As will be clear from the disclosure herein, the use of natural or synthetic analogs, mutants, variants, alleles, homologs, and orthologs (herein collectively referred to as "analogs") of the nanobodies of the present disclosure as defined herein, and in particular analogs of the nanobodies of SEQ ID NOs: 1-20, is also within the scope of the invention. Thus, according to one embodiment of the present disclosure, the term "nanobody of the present disclosure" in its broadest sense also encompasses such analogs.

Generally, in such analogs, one or more amino acid residues may have been replaced, deleted, and/or added, compared to the nanobodies of the present disclosure as defined herein. Such substitutions, insertions or deletions may be made in one or more of the framework regions and/or in one or more of the CDRs. When such substitutions, insertions or deletions are made in one or more of the framework regions, they may be made at one or more of the Hallmark residues and/or at one or more of the other positions in the framework residues, although substitutions, insertions or deletions at the Hallmark residues are generally less preferred (unless these are suitable humanizing substitutions as described herein).

Yet another modification may comprise the introduction of one or more detectable labels or other signal-generating groups or moieties, depending on the intended use of the labelled nanobody. Suitable labels and techniques for attaching to, using and detecting nanobodies will be clear to the skilled person, and for example include, but are not limited to, fluorescent labels (such as fluorescein, isothiocyanate, rhodamine, phycoerythrin, phycocyanin, allophycocyanin, o-phthaldehyde, and fluorescamine and fluorescent metals such as ¹⁵²Eu or others metals from the lanthanide series), phosphorescent labels, chemiluminescent labels or bioluminescent labels (such as luminol, isoluminol, theromatic acridinium ester, imidazole, acridinium salts, oxalate ester, dioxetane or GFP and its analogs), radio-isotopes (such as ³H,¹²⁵I, ³²P, ³⁵S, ¹⁴C, ⁵¹Cr, ³⁶Cl, ⁵⁷Co, ⁵⁸Co, ⁵⁹Fe, and ⁷⁵Se), metals, metal chelates or metal cations (for example metal cations such as ^{99m}Tc, ¹²³I, ¹¹¹In, ¹³¹I, ⁹⁷Ru, ⁶⁷Cu, ⁶⁷Ga, and ⁶⁸Ga or other metals or metal cations that are particularly suited for use in *in vivo*, *in vitro* or *in situ* diagnosis and imaging, such as (¹⁵⁷Gd, ⁵⁵Mn, ¹⁶²Dy, ⁵²Cr, and ⁵⁶Fe), as well as chromophores and enzymes (such as malate dehydrogenase, staphylococcal nuclease, delta-V-steroid isomerase, yeast alcohol dehydrogenase, alpha-glycerophosphate dehydrogenase, triosephosphate isomerase, biotin peroxidase, horseradish peroxidase, alkaline phosphatase, asparaginase, glucose oxidase, β-galactosidase, ribonuclease, urease, catalase, glucose-VI-phosphate dehydrogenase, glucoamylase and acetylcholine esterase). Other suitable labels will be clear to the skilled person, and for example include moieties that may be detected using NMR or ESR spectroscopy.

Such labelled nanobodies and polypeptides of the present disclosure may for example be used for in vitro, *in vivo* or in situ assays (including immunoassays known per se such as ELISA, RIA, EIA and other "sandwich assays", etc.) as well as *in vivo* diagnostic and imaging purposes, depending on the choice of the specific label.

As will be clear to the skilled person, another modification may involve the introduction of a chelating group, for example to chelate one of the metals or metal cations referred to above. Suitable chelating groups for example include, without limitation, diethylenetriaminepentaacetic acid (DTPA) or ethylenediaminetetraacetic acid (EDTA).

Yet another modification may comprise the introduction of a functional group that is part of a specific binding pair, such as the biotin-(strept)avidin binding pair. Such a functional group may be used to link the nanobody of the present disclosure to another protein, polypeptide, or chemical compound that is bound to the other half of the binding pair, i.e., through formation of the binding pair. For example, a nanobody of the present disclosure may be conjugated to biotin and linked to another protein, polypeptide, compound, or carrier conjugated to avidin or streptavidin. For example, such a conjugated nanobody may be used as a reporter, for example in a diagnostic system where a detectable signal-producing agent is conjugated to avidin or streptavidin. Such binding pairs may, for example, also be used to bind the nanobody of the present disclosure to a carrier, including carriers suitable for pharmaceutical purposes. One non-limiting example is the case with liposomal formulations described by Cao and Suresh, Journal of Drug Targeting, 8, 4, 257 (2000). Such binding pairs may also be used to link a therapeutically active agent to the nanobody of the present disclosure.

Other potential chemical and enzymatic modifications will be clear to the skilled person. Such modifications may also be introduced for research purposes (e.g., to study function-activity relationships). Reference is for example, made to Lundblad and Bradshaw, Biotechnol. Appl. Biochem., 26, 143-151 (1997).

As mentioned above, the present disclosure also relates to proteins or polypeptides that essentially consist of at least one nanobody of the present disclosure. By "essentially consist of" is meant that the amino acid sequence of the polypeptide of the present disclosure is either completely identical to the amino acid sequence of the nanobody of the present disclosure or corresponds to the amino acid sequence of the nanobody of the present disclosure which has a limited number of amino acid residues, such as 1-20 ammo acid residues, for example 1-10 amino acid residues and preferably 1-6 amino acid residues, such as 1, 2, 3, 4, 5 or 6 amino acid residues, added at the amino terminus, at the carboxy terminus, or at both the amino terminus and the carboxy terminus of the amino acid sequence of the nanobody.

Said amino acid residues may or may not change, alter or otherwise influence the (biological) properties of the nanobody and may or may not add further functionality to the nanobody.

According to another embodiment, the polypeptide of the present disclosure comprises a nanobody of the present disclosure, which is fused at its amino terminus, at its carboxy terminus, or at both its amino terminus and at its carboxy terminus to at least one further amino acid sequence, i.e. so as to provide a fusion protein comprising said nanobody of the present disclosure and the one or more further amino acid sequences. Such a fusion will also be referred to herein as a "nanobody fusion".

One or more further amino acid sequences may be any suitable and/or desired amino acid sequences. The further amino acid sequence(s) may or may not change, alter, or otherwise influence the (biological) properties of the nanobody, and may or may not add further functionality to the nanobody or the polypeptide of the present disclosure. Preferably, the further amino acid sequence is such that it confers one or more desired properties or functionalities to the nanobody or the polypeptide of the present disclosure.

A nucleic acid of the present disclosure may be in the form of single or double stranded DNA or RNA, and is preferably in the form of double stranded DNA. For example, the nucleotide sequences of the present disclosure may be genomic DNA, cDNA, or synthetic DNA (such as DNA with a codon usage that has been specifically adapted for expression in an intended host cell or host organism).

According to one embodiment of the present disclosure, the nucleic acid of the present disclosure is in essentially isolated from, as defined herein.

The nucleic acid of the present disclosure may also be in the form of, be present in, and/or be part of a vector, such as, for example a plasmid, a cosmid, or a YAC, which again may be in essentially isolated form.

The nucleic acid of the present disclosure may also be in the form of, be present in, and/or be part of a genetic construct, as will be clear to the person skilled in the art. Such a genetic construct generally comprises at least one nucleic acid of the present disclosure that is optionally linked to one or more elements of a genetic construct known *per se*, such as, for example, one or more suitable regulatory elements (such as a suitable promoter(s), enhancer(s), terminator(s), etc.) and the further elements of the genetic construct mentioned herein. Such a genetic construct comprising at least one nucleic acid of the present disclosure will also be referred to herein as a "genetic construct of the present disclosure".

The genetic construct of the present disclosure may be DNA or RNA, and is preferably double-stranded DNA. The genetic construct of the present disclosure may also be in a form suitable for transformation into an intended host cell or host organism, in a form suitable for integration into the genomic DNA of the intended host cell, or in a form suitable for independent replication, maintenance, and/or inheritance in the intended host organism. For instance, the genetic construct of the present disclosure may be in the form of a vector, such as for example a plasmid, cosmid, YAC, a viral vector, or transposon. In particular, the vector may be an expression vector, i.e. a vector that may provide for expression in vitro and/or *in vivo* (e.g., in a suitable host cell, host organism and/or expression system).

In a preferred but non-limiting embodiment, the genetic construct of the present disclosure comprises a) at least one nucleic acid of the present disclosure; operably linked to b) one or more regulatory elements, such as a promoter and optionally a suitable terminator; and optionally also c) one or more further elements of the genetic construct known *per se*; in which the terms "regulatory element," "promoter," "terminator," and "operably linked" have their usual meaning in the art (as further described herein); and in which said "further elements" present in the genetic construct may for example be 3'- or 5'-UTR sequences, leader sequences, selection markers, expression markers/reporter genes, and/or elements that may facilitate or increase (the efficiency of) transformation or integration. These and other suitable elements for such s genetic construct will be clear to the skilled person, and may for instance, depend upon the type of construct used, the intended host cell or host organism; the manner in which the nucleotide sequences of the present disclosure of interest are to be expressed (e.g. via constitutive, transient or inducible expression); and/or the transformation technique to be used. For example, regulatory sequences, promoters, and terminators known per se for the expression and production of antibodies and antibody fragments (including but not limited to (single) domain antibodies and ScFv fragments) may be used in a substantially similar manner.

Preferably, in the genetic construct of the present disclosure, said at least one nucleic acid of the present disclosure and said regulatory elements, and optionally said one or more further elements, are "operably linked" to each other, by which is generally meant that they are in a functional relationship with each other. For instance, a promoter is considered "operably linked" to a coding sequence if said promoter is able to initiate or otherwise control/regulate the transcription and/or the expression of the coding sequence (in which said coding sequence should be understood as being "under the control of said promotor). Generally, when two nucleotide sequences are operably linked, they will be in the same orientation and usually also in the same reading frame. They will usually also be essentially contiguous, although this may not be required.

Preferably, the regulatory elements and further elements of the genetic construct of the present disclosure are such that they are capable of providing their intended biological function in the intended host cell or host organism.

For instance, a promoter, enhancer, or terminator should be "operable" in the intended host cell or host organism, by which is meant that (for example) said promoter should be capable of initiating or otherwise controlling/regulating the transcription and/or the expression of a nucleotide sequence - e.g., a coding sequence - to which it is operably linked (as defined herein).

Some particularly preferred promoters include, but are not limited to, promoters known per se for the expression in the host cells mentioned herein; and in particular promoters for the expression in the bacterial cells, such as those mentioned herein and/or those used in the Examples.

A selection marker should be such that it allows - i.e., under appropriate selection conditions - host cells and/or host organisms that have been (successfully) transformed with the nucleotide sequence of the present disclosure to be distinguished from host cells/organisms that have not been (successfully) transformed. Some preferred, but non-limiting examples of such markers are genes that provide resistance to antibiotics (such as kanamycin or ampicillin), genes that provide temperature resistance, or genes that allow the host cell or host organism to be maintained in the absence of certain factors, compounds, and/or (food) components in the medium that are essential for the survival of non-transformed cells or organisms.

A leader sequence should be such that - in the intended host cell or host organism - it allows for the desired post-translational modifications and/or such that it directs the transcribed mRNA to a desired part or organelle of a cell. A leader sequence may also allow for secretion of the expression product from said cell. As such, the leader sequence may be any pro-, pre-, or prepro-sequence operable in the host cell or host organism. Leader sequences may not be required for expression in a bacterial cell. For example, leader sequences known per se for the expression and production of antibodies and antibody fragments (including but not limited to single domain antibodies and ScFv fragments) may be used in a substantially similar manner.

An expression marker or reporter gene should be such that - in the host cell or host organism - it allows for detection of the expression of (a gene or nucleotide sequence present on) the genetic construct. An expression marker may optionally also allow for the localization of the expressed product, e.g., in a specific part or organelle of a cell and/or in (a) specific cell(s), tissue(s), organ(s), or part(s) of a multicellular organism. Such reporter genes may also be expressed as a protein fusion with the amino acid sequence of the present disclosure. Some preferred, but non-limiting examples include fluorescent proteins such as GFP.

Some preferred, but non-limiting examples of suitable promoters, terminators, and further elements include those that are useful for the expression in the host cells mentioned herein, and in particular those that are suitable for expression in bacterial cells, such as those mentioned herein and/or those used in the Examples below. For some (further) non-limiting examples of the promoters, selection markers, leader sequences, expression markers and further elements that may be present/used in the genetic construct of the present disclosure - such as terminators, transcriptional and/or translational enhancers and/or integration factors -reference is made to the general handbooks such as Sambrook et al. and Ausubel et al. mentioned above, as well as to the examples given in WO 95/07463, WO 96/23810, WO 95/07463, WO 95/21191, WO 97/11094, WO 97/42320, WO 98/06737, WO 98/21355, US- A-6,207,410, US-A-5,693,492 and EP 1 085 089. Other examples will be clear to the skilled person. Reference is also made to the general background art cited above and further references cited herein.

The genetic construct of the present disclosure may generally be provided by suitably linking the nucleotide sequence(s) of the present disclosure to one or more further elements described above, for example, using the techniques described in the general handbooks such as Sambrook et al. and Ausubel et al., mentioned above.

Typically, the genetic construct of the present disclosure will be obtained by inserting the nucleotide sequence of the present disclosure into a suitable (expression) vector known per se. Some preferred, but non-limiting examples of suitable expression vectors are those used in the following Examples below, as well as those mentioned herein.

The nucleic acids of the present disclosure and/or the genetic construct of the present disclosure may be used to transform a host cell or host organism, i.e., for expression and/or production of the nanobody or polypeptide of the present disclosure. Suitable hosts or host cells will be clear to the skilled person, and may, for example, be any suitable fungal, prokaryotic, or eukaryotic cell or cell line or any suitable fungal, prokaryotic, or eukaryotic organism.

Generally, for the prevention and/or treatment of the diseases and disorders mentioned herein and depending on the specific disease or disorder to be treated, the potency of the specific nanobody and polypeptide of the present disclosure to be used, the specific route of administration and the specific pharmaceutical formulation or composition to be used, the nanobodies and polypeptides of the present disclosure will generally be administered in an amount between 1 gram and 0.01 microgram per kg body weight per day, preferably between 0.1 gram and 0.1 microgram per kg body weight per day, such as about 1, 10, 100 or 1000 microgram per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, clinicians may choose to deviate from these amounts, for example, on the basis of the factors cited above and their expert judgment. Generally, some guidance on the amounts to be administered may be obtained from the amounts usually administered for comparable conventional antibodies or antibody fragments directed against the same target administered via essentially the same route, taking into account however differences in affinity/avidity, efficacy, biodistribution, half-life and similar factors well known to the skilled person.

It should also be noted that, when the nanobodies of the present disclosures contain one or more other CDR sequences than the preferred CDR sequences mentioned above, these CDR sequences may be obtained in any manner known per se, for example from nanobodies (preferred), V_{H} domains from conventional antibodies (and in particular from human antibodies), heavy chain antibodies, conventional 4-chain antibodies (such as conventional human 4-chain antibodies) or other immunoglobulin sequences directed against A-β. Such immunoglobulin sequences directed against A-β may be generated in any manner known per se, as will be clear to the skilled person, i.e., by immunization with A-β or by screening a suitable library of immunoglobulin sequences with A-β, or any suitable combination thereof. Optionally, this may be followed by techniques such as random or site-directed mutagenesis and/or other techniques for affinity maturation known *per se*. Suitable techniques for generating such immunoglobulin sequences will be clear to the skilled person, and for example, include the screening techniques reviewed by Hoogenboom, Nature Biotechnology, 23, 9, 1105-1116 (2005). Other techniques for generating immunoglobulins against a specified target include for example the Nanoclone technology (as for example described in the non-prepublished US provisional patent application 60/648,922), so-called SLAM technology (as for example described in the European patent application 0 542 810), the use of transgenic mice expressing human immunoglobulins or the well-known hybridoma techniques (see for example Larrick et al, Biotechnology, Vol.7, 1989, p. 934). All these techniques are useful to generate immunoglobulins against A-β, and the CDRs of such immunoglobulins are useful in the nanobodies of the present disclosure, i.e. as outlined above. For example, the sequences of such CDRs may be determined, synthesized and/or isolated, and inserted into the sequence of a nanobody of the present disclosure (e.g. so as to replace the corresponding native CDRs), all using techniques known per se such as those described herein, or nanobodies of the present disclosure containing such CDRs (or nucleic acids encoding the same) may be synthesized de novo, again using the techniques mentioned herein.

The present disclosure will now be further described by means of the following non-limiting examples and accompanying drawings.

### EXAMPLES

### Example 1 Construction of the Alpaca Immune Library

### 1.1 Alpaca Immunization

Antigens used for immunization, Human B7-H3-Fc (ACRO B73-H5253), Human B7-H3-His (ACRO B73-H52E2), and Mouse B7-H3-Fc (ACRO B73-M5255), were all commercially available. Immunization was performed by subcutaneous injection, and a total of 2 alpacas, NM018 and NM019, were immunized. NM018 was alternately immunized with human antigens Human B7-H3-Fc and Human B7-H3-His; NM019 was alternately immunized with human and mouse antigens Human B7-H3-Fc and Mouse B7-H3-Fc. The single immunization dose was 500 µg. Freund's incomplete adjuvant was used for primary immunization, and Freund's complete adjuvant was used for subsequent immunizations. Immunization was performed every two weeks for a total of 5 immunizations.

### 1.2 Determination of Serum Titer

After the 4th and 5th immunizations, blood was collected to determine the titer of antibodies targeting the antigen Human B7-H3-Fc in the serum. The specific detection method was as follows:

The antigen Human B7-H3-Fc was diluted with PBS to a final concentration of 2 µg/mL, and 30 µL of the dilution was added to the first ELISA plate which was coated overnight at 4°C. On the day of the immune titer assay, the plate was rinsed three times with PBST, then blocked with PBSM containing 5% skim milk at room temperature for two hours, and then rinsed three times with PBST. The unimmunized negative serum and the serum after 4th or 5th immunization were diluted with PBS on another plate. The first well was diluted 2000-fold, and the subsequent 7 wells were diluted in 2-fold serial dilutions. The diluted sera were added to the first ELISA plate and incubated for 1 h at room temperature. After washing the plate three times with PBST, a secondary antibody Goat anti-Llama IgG (H + L) Secondary Antibody [HRP] (purchased from NOVUS Cat. NBP1-75088) was added at 1:5000 and incubated at room temperature for 0.5 h. After completion of incubation, the plates were washed six times with PBST and developed by adding TMB (SurModics, TMBS-1000-01). Based on the development results, 2M HCl was added to stop the reaction, and the plate was read at OD450 by a microplate reader (Molecular Devices, SpecterMax 190).

Results are shown in Table 1. After five immunizations, the titers of antibodies targeting the antigen Human B7-H3-Fc in the sera of two alpacas reached a dilution ratio of 1:32K or higher.

**Table 1. Determination of serum IgG titer of alpaca immunized with B7-H3**

| Sample | Negative serum | | Serum after 4th immunization | | Serum after 5th immunization | |
|---|---|---|---|---|---|---|
| Dilution /Animal number | NM018 | NM019 | NM018 | NM019 | NM018 | NM019 |
| 1:2K | 0.37 | 0.37 | 1.81 | 1.75 | 1.78 | 1.72 |
| 1:4K | 0.36 | 0.36 | 1.74 | 1.53 | 1.71 | 1.49 |
| 1:8K | 0.34 | 0.33 | 1.56 | 1.25 | 1.54 | 1.19 |
| 1:16K | 0.35 | 0.34 | 1.3 | 0.9 | 1.34 | 0.81 |
| 1:32K | 0.32 | 0.31 | 0.82 | 0.56 | 0.91 | 0.56 |
| 1:64K | 0.33 | 0.32 | 0.62 | 0.46 | 0.64 | 0.43 |
| 1:128K | 0.33 | 0.32 | 0.48 | 0.41 | 0.5 | 0.39 |
| 1:256K | 0.35 | 0.36 | 0.46 | 0.4 | 0.45 | 0.39 |

### Example 2 Construction of Phage Display Library and Screening of B7-H3-targeting Nanobodies

Using phage display technology, the antibody genes from peripheral blood cells (PBMCs) of alpaca immunized with B7-H3 antigen protein were cloned into phage display vectors to construct an antibody library. The library was screened using Human B7-H3-Fc, Human B7-H3-His, and Mouse B7-H3-Fc as screening antigens, resulting in the identification of multiple nanobodies specifically binding to the B7-H3 protein.

### 2.1 Construction of Phage Display Library of Alpaca-Derived Nanobodies

100 mL of blood was collected from alpacas to isolate PBMCs. RNA was extracted from PBMCs by the chloroform method and reverse transcribed into cDNA. Degenerate primers were designed based on the germline situations of VHH antibodies. DNA fragments encoding VHH-CH2 were obtained by PCR amplification and agarose gel electrophoresis recovery of the PCR products. Then, the DNA fragments encoding the variable domain (VHH) were amplified by a secondary PCR using the DNA fragments encoding VHH-CH2 as a template. Then, the DNA fragments encoding VHH were purified by enzymatic digestion and constructed into a phage display vector. Finally, the VHH-expressing vector was transformed into competent *E. coli* by an electroporator. 10 µL of transformants were spread on a plate for a single colony used for sequencing. The capacity of the antibody library and the correct insertion rate of antibody genes were determined by the dilution-plating method and monoclonal sequencing analysis, respectively. The capacity of the single domain antibody library was determined to be in the order of magnitude of 10⁸, and the sequencing analysis showed that the correct insertion rate of the antibody genes was greater than 80%.

### 2.2 Screening of the Phage Display Library of the Antibody Gene

The phage display library was screened with immunotubes and a magnetic bead sorter. Three rounds of screening were performed by cross-screening with Human B7-H3-Fc, Human B7-H3-His, and Mouse B7-H3-Fc.

### 2.2.1 Screening of Phage Display Library of Antibody Genes by the Immunotube Method

The screening principle of the immunotube method is as follows. The antigen protein Human B7-H3-Fc or Human B7-H3-His is coated on the surface of the immunotube with a high adsorptive capacity. The phage display library of antibodies is added to the immunotube, and incubated with the antigen protein adsorbed on the surface of the immunotube, followed by a washing and eluting the antibodies. Specific monoclonal antibodies against the antigen are enriched through this panning process.

The specific method is as follows. 1 mL of 300 µg/mL Human B7-H3-Fc or Human B7-H3-His was added to immunotubes in different groups and coated at 4°C. The phage suspension was incubated with the protein-coated immunotube, and binding and washing were performed according to the method of the immunotube screening system. Thereafter, the phage was eluted with Trypsin, and the eluted phage solution was thoroughly mixed with *E. coli* SS320 cells at logarithmic phase, incubated statically at 37°C for 30 minutes. The mixture was then spread in 2YT medium, and cultured in an incubator at 37°C overnight for the next round of screening. Simultaneously, the eluted phage solution was serially diluted 10-fold with SS320 cells at logarithmic phase. The dilutions were incubated at 37°C for 30 minutes, mixed well, and 2 µL was dropped onto a plate and cultured in an incubator at 37°C overnight for detection.

### 2.2.2 Screening of Phage Display Library of Antibody Genes by Magnetic Bead Method (Kingfisher Method)

Human B7-H3-Fc and Human B7-H3-His were labeled with biotin, then bound to Dynabeads magnetic beads. The antigen-bound magnetic beads were incubated with the antibody gene phage display library, followed by washing and elution steps, thereby specific monoclonal antibodies against the antigen were significantly enriched through this panning process.

The specific method was as follows. The phage suspension of the prepared alpaca immune library was diluted and blocked with 5% BSA, and co-incubated with Dynabeads, and the phages after negative screening incubation were collected. The magnetic beads were bound and washed with Human B7-H3-Fc or Human B7-H3-His according to the Kingfisher Magnetic Bead Screening System method, and 5% BSA was incubated with the magnetic beads. The phage suspension collected after negative screening was incubated with Dynabeads coated with biotin-labeled antigen and blocked, followed by binding and washing according to the Kingfisher magnetic bead screening system method. The phage was eluted with Trypsin, and the eluted phage solution was thoroughly mixed with *E. coli* SS320 cells at logarithmic phase, incubated statically at 37°C for 30 minutes. The mixture was then spread in 2YT medium, and cultured in an incubator at 37°C overnight for the next round of screening. Simultaneously, the eluted phage solution was diluted 10-fold with SS320 cells at logarithmic phase. The dilutions were incubated at 37°C for 30 minutes, mixed well, and 2 µL of them was dropped on a plate and cultured in an incubator at 37°C overnight for detection.

### 2.2.3 Panning of Single Clones

The phage pools obtained from each round of elution from the immunotube screening and the magnetic bead screening methods were serially diluted 5-fold with 5% PBSM and screened by ELISA.

2 µg/mL Human B7-H3-Fc or Mouse B7-H3-Fc was added to a 96-well plate, 30 µL per well, and coated at 4°C overnight. The plate was blocked with 5% PBSM at room temperature for 1 h. 30 µL/well of the serially-diluted VHH or Phage expression supernatant obtained from 2.2.1 and 2.2.2 was added, and incubated at room temperature for 1 h. VHH expression supernatant was added with 1:5000 diluted secondary antibody Anti-Flag-HRP (Sigma Cat H7425-1VL) at 30 µL/well, and the Phage expression supernatant was added with 1:20000 diluted secondary antibody Anti-M13-HRP (SinoBiological Cat 11973-MM05T) at 30 µL/well, for 1 h at room temperature. TMB was added for color development for 5 to 20 min, after which the reaction was stopped by adding a stop solution, and the data was read at OD450 by a microplate reader.

A large number of single clones were picked for ELISA primary screening. After sequencing analysis in combination with ELISA, 33 nanobodies with sequence diversity that bind to the B7-H3 antigen were obtained in the primary screening. 33 sequences were selected for ELISA affinity redetermination. The results are shown in FIG. 1.

Finally, based on affinity and mouse cross-reactivity, 13 monoclonal antibodies were finally selected for the construction of expression vectors for the full-length sequence (VHH and Fc fusion protein), as detailed in Table 2.

**Table 2. Amino acid sequences of VHH and VHH-Fc antibodies**

| | VHH sequence | Full-length sequence (VHH + Fc) |
|---|---|---|
| C23 | EVQVVESGGDLVQP GGSLRLSCAASGFIIS TYSMSWVRQAPGK GLEWVSDINSGGGS TYYADSVKGRFTISR DNAKNTLYLQMNSL KPEDTAVYYCARRT | EVQVVESGGDLVQPGGSLRLSCAASGFIISTYSMSWVR QAPGKGLEWVSDINSGGGSTYYADSVKGRFTISRDNA KNTLYLQMNSLKPEDTAVYYCARRTQRTYYSGNYYG GGMDYWGKGTLVTVSSEPKSSDKTHTCPPCPAPELLG GPSVFLFPPKPKDTLMISRTPEVTCVVVDVSHEDPEVK FNWYVDGVEVHNAKTKPREEQYNSTYRVVSVLTVLH QDWLNGKEYKCKVSNKALPAPIEKTISKAKGQPREPQ |
| | QRTYYSGNYYGGG MDYWGKGTLVTVS S (SEQ ID NO: 1) | VYTLPPSRDELTKNQVSLTCLVKGFYPSDIAVEWESNG QPENNYKTTPPVLDSDGSFFLYSKLTVDKSRWQQGNV FSCSVMHEALHNHYTQKSLSLSPGK (SEQ ID NO: 70) |
| A13 | QLQLVESGGGLVQA GGSLRLSCAASGSTS SNHAVGWYRQVPG KQRESVASIDSGGST YYVDFVKGRFTISRD KNTLYLQMNSLKPE DTAVYYCYLRTWR GDLYWGQGTQVTV SS (SEQ ID NO: 2) | QLQLVESGGGLVQAGGSLRLSCAASGSTSSNHAVGWY RQVPGKQRESVASIDSGGSTYYVDFVKGRFTISRDKNT LYLQMNSLKPEDTAVYYCYLRTWRGDLYWGQGTQV TVSSEPKSSDKTHTCPPCPAPELLGGPSVFLFPPKPKDT LMISRTPEVTCVVVDVSHEDPEVKFNWYVDGVEVHN AKTKPREEQYNSTYRVVSVLTVLHQDWLNGKEYKCK VSNKALPAPIEKTISKAKGQPREPQVYTLPPSRDELTKN QVSLTCLVKGFYPSDIAVEWESNGQPENNYKTTPPVLD SDGSFFLYSKLTVDKSRWQQGNVFSCSVMHEALHNH YTQKSLSLSPGK (SEQ ID NO: 71) |
| A2 | QVQLVESGGGLVQA GGSLRLTCAASGRTF SSNNMGWFRQAPG KEREFVTATKWSSG VLYYADSADSVKGR FTNSRDVARNTVYL HMTNLKPEDTAVYY CAAMSRVAAMSRL VGDYDYWGQGTLV TVSS (SEQ ID NO: 3) | QVQLVESGGGLVQAGGSLRLTCAASGRTFSSNNMGW FRQAPGKEREFVTATKWSSGVLYYADSADSVKGRFTN SRDVARNTVYLHMTNLKPEDTAVYYCAAMSRVAAM SRLVGDYDYWGQGTLVTVSSEPKSSDKTHTCPPCPAP ELLGGPSVFLFPPKPKDTLMISRTPEVTCVVVDVSHEDP EVKFNWYVDGVEVHNAKTKPREEQYNSTYRVVSVLT VLHQDWLNGKEYKCKVSNKALPAPIEKTISKAKGQPR EPQVYTLPPSRDELTKNQVSLTCLVKGFYPSDIAVEWE SNGQPENNYKTTPPVLDSDGSFFLYSKLTVDKSRWQQ GNVFSCSVMHEALHNHYTQKSLSLSPGK (SEQ ID NO: 72) |
| A3 | EVQLVESGGGLVQA GGSLRLSCAASGPGF SRYSVGWFRQTPGK EREFVATIMWLGDT TYYADSVKGRFTISR DNAKDTAYLQMNS LKPEDTAVYYCAVK TSLRQYTNRDEYDY WGQGTQVTVSS (SEQ ID NO: 4) | EVQLVESGGGLVQAGGSLRLSCAASGPGFSRYSVGWF RQTPGKEREFVATIMWLGDTTYYADSVKGRFTISRDN AKDTAYLQMNSLKPEDTAVYYCAVKTSLRQYTNRDE YDYWGQGTQVTVSSEPKSSDKTHTCPPCPAPELLGGPS VFLFPPKPKDTLMISRTPEVTCVVVDVSHEDPEVKFNW YVDGVEVHNAKTKPREEQYNSTYRVVSVLTVLHQDW LNGKEYKCKVSNKALPAPIEKTISKAKGQPREPQVYTL PPSRDELTKNQVSLTCLVKGFYPSDIAVEWESNGQPEN NYKTTPPVLDSDGSFFLYSKLTVDKSRWQQGNVFSCS VMHEALHNHYTQKSLSLSPGK (SEQ ID NO: 73) |
| A77 | QVQLVESGGGLVQP GGSLRLSCAASGFTF SSYDMSWYRQAPG KERELVAFITSAGSS TNYADSVKGRFTISR DNAKNTLYLQMNSL KPEDTAVYYCNAEG AYYSGNYYYTMHD YWGQGTQVTVSS (SEQ ID NO: 5) | QVQLVESGGGLVQPGGSLRLSCAASGFTFSSYDMSWY RQAPGKERELVAFITSAGSSTNYADSVKGRFTISRDNA KNTLYLQMNSLKPEDTAVYYCNAEGAYYSGNYYYTM HDYWGQGTQVTVSSEPKSSDKTHTCPPCPAPELLGGPS VFLFPPKPKDTLMISRTPEVTCVVVDVSHEDPEVKFNW YVDGVEVHNAKTKPREEQYNSTYRVVSVLTVLHQDW LNGKEYKCKVSNKALPAPIEKTISKAKGQPREPQVYTL PPSRDELTKNQVSLTCLVKGFYPSDIAVEWESNGQPEN NYKTTPPVLDSDGSFFLYSKLTVDKSRWQQGNVFSCS VMHEALHNHYTQKSLSLSPGK (SEQ ID NO: 74) |
| A83 | EVQLQESGGGLVQP GGSLRLSCVASGNIF SINAMGWYRQGPGK QRELVAALSSGGRT YYIGSVKGRFTISRD NAKNTLYLQMDSLK PEDTAIYYCAAVNS GSYYSDDYRGQGTQ VTVSS (SEQ ID NO: 6) | EVQLQESGGGLVQPGGSLRLSCVASGNIFSINAMGWY RQGPGKQRELVAALSSGGRTYYIGSVKGRFTISRDNAK NTLYLQMDSLKPEDTAIYYCAAVNSGSYYSDDYRGQG TQVTVSSEPKSSDKTHTCPPCPAPELLGGPSVFLFPPKP KDTLMISRTPEVTCVVVDVSHEDPEVKFNWYVDGVEV HNAKTKPREEQYNSTYRVVSVLTVLHQDWLNGKEYK CKVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEALH NHYTQKSLSLSPGK (SEQ ID NO: 75) |
| B102 | EVQLVESGGGLVQP GGSLRLSCAASGNIF SIAGMGWHRQAPGK QREWVATITRGGRIT YADSVKGRFTISRDI AENTVYLQMRSLNP EDTAVYYCTTSGYW GQGTQVTVSS (SEQ ID NO: 7) | EVQLVESGGGLVQPGGSLRLSCAASGNIFSIAGMGWH RQAPGKQREWVATITRGGRITYADSVKGRFTISRDIAE NTVYLQMRSLNPEDTAVYYCTTSGYWGQGTQVTVSS EPKSSDKTHTCPPCPAPELLGGPSVFLFPPKPKDTLMIS RTPEVTCVVVDVSHEDPEVKFNWYVDGVEVHNAKTK PREEQYNSTYRVVSVLTVLHQDWLNGKEYKCKVSNK ALPAPIEKTISKAKGQPREPQVYTLPPSRDELTKNQVSL TCLVKGFYPSDIAVEWESNGQPENNYKTTPPVLDSDGS FFLYSKLTVDKSRWQQGNVFSCSVMHEALHNHYTQK SLSLSPGK (SEQ ID NO: 76) |
| B91 | EVQLQESGGGLVQP GGSLRLSCAASGFTF SSYAMSWYRQVPG KERELIADISSAGDS TNYIDSVKGRFTISR DNAKNTVYLQMNS LKPEDTAVYYCNAE GAYYNGNYYYTMH DYWGQGTQVTVSS (SEQ ID NO: 8) | EVQLQESGGGLVQPGGSLRLSCAASGFTFSSYAMSWY RQVPGKERELIADISSAGDSTNYIDSVKGRFTISRDNAK NTVYLQMNSLKPEDTAVYYCNAEGAYYNGNYYYTM HDYWGQGTQVTVSSEPKSSDKTHTCPPCPAPELLGGPS VFLFPPKPKDTLMISRTPEVTCVVVDVSHEDPEVKFNW YVDGVEVHNAKTKPREEQYNSTYRVVSVLTVLHQDW LNGKEYKCKVSNKALPAPIEKTISKAKGQPREPQVYTL PPSRDELTKNQVSLTCLVKGFYPSDIAVEWESNGQPEN NYKTTPPVLDSDGSFFLYSKLTVDKSRWQQGNVFSCS VMHEALHNHYTQKSLSLSPGK (SEQ ID NO: 77) |
| C184 | QVQLVESGGGLVQP GGSLRLSCAASGFTF SSYGLSWVRQAPGK GLEWVSDINSGGGS TLYSDSVTGRFTISR DNAKNTVYLQMNS LKPEDTAVYYCAKV VQGAGTWYWDRYL EVWGQGTLVTVSS (SEQ ID NO: 9) | QVQLVESGGGLVQPGGSLRLSCAASGFTFSSYGLSWV RQAPGKGLEWVSDINSGGGSTLYSDSVTGRFTISRDNA KNTVYLQMNSLKPEDTAVYYCAKVVQGAGTWYWDR YLEVWGQGTLVTVSSEPKSSDKTHTCPPCPAPELLGGP SVFLFPPKPKDTLMISRTPEVTCVVVDVSHEDPEVKFN WYVDGVEVHNAKTKPREEQYNSTYRVVSVLTVLHQD WLNGKEYKCKVSNKALPAPIEKTISKAKGQPREPQVY TLPPSRDELTKNQVSLTCLVKGFYPSDIAVEWESNGQP ENNYKTTPPVLDSDGSFFLYSKLTVDKSRWQQGNVFS CSVMHEALHNHYTQKSLSLSPGK (SEQ ID NO: 78) |
| C357 | EVQLVESGGGLVQA GGSLGLSCVASIDTF TLNAISWYRQAPGK PRELVARSWRSGDT NYADSVKGRFTISV DNSKKTVYLQMNSL EPEDTAVYYCNAIR GADDFWGQGTQVT VSS (SEQ ID NO: 10) | EVQLVESGGGLVQAGGSLGLSCVASIDTFTLNAISWYR QAPGKPRELVARSWRSGDTNYADSVKGRFTISVDNSK KTVYLQMNSLEPEDTAVYVCNAIRGADDFWGQGTQV TVSSEPKSSDKTHTCPPCPAPELLGGPSVFLFPPKPKDT LMISRTPEVTCVVVDVSHEDPEVKFNWYVDGVEVHN AKTKPREEQYNSTYRVVSVLTVLHQDWLNGKEYKCK VSNKALPAPIEKTISKAKGQPREPQVYTLPPSRDELTKN QVSLTCLVKGFYPSDIAVEWESNGQPENNYKTTPPVLD SDGSFFLYSKLTVDKSRWQQGNVFSCSVMHEALHNH YTQKSLSLSPGK (SEQ ID NO: 79) |
| C59 | EVQVVESGGGLVQP GGSLRLSCAASGFTF SSYDMSWYRQAPG KERELVAFITSAGGS TNYADSVKGRFTISR DNAKNTLYLQMNSL KPEDTAVYYCNAEG AYYNGNYYYTMHD YWGQGTQVTVSS (SEQ ID NO: 11) | EVQVVESGGGLVQPGGSLRLSCAASGFTFSSYDMSWY RQAPGKERELVAFITSAGGSTNYADSVKGRFTISRDNA KNTLYLQMNSLKPEDTAVYYCNAEGAYYNGNYYYT MHDYWGQGTQVTVSSEPKSSDKTHTCPPCPAPELLGG PSVFLFPPKPKDTLMISRTPEVTCVVVDVSHEDPEVKF NWYVDGVEVHNAKTKPREEQYNSTYRVVSVLTVLHQ DWLNGKEYKCKVSNKALPAPIEKTISKAKGQPREPQV YTLPPSRDELTKNQVSLTCLVKGFYPSDIAVEWESNGQ PENNYKTTPPVLDSDGSFFLYSKLTVDKSRWQQGNVF SCSVMHEALHNHYTQKSLSLSPGK (SEQ ID NO: 80) |
| D16 | QVQLQESGGGLVQA GGSLRLSCAASIPDF SRYSVGWFRQTPGK EREFVATIMWLGDT TYYADSVKGRFTISR DNAKDTAYLQMNS LKPEDTAVYYCAVK TSLRQYTNPNEYDY WGQGTQVTVSS (SEQ ID NO: 12) | QVQLQESGGGLVQAGGSLRLSCAASIPDFSRYSVGWF RQTPGKEREFVATIMWLGDTTYYADSVKGRFTISRDN AKDTAYLQMNSLKPEDTAVYYCAVKTSLRQYTNPNE YDYWGQGTQVTVSSEPKSSDKTHTCPPCPAPELLGGPS VFLFPPKPKDTLMISRTPEVTCVVVDVSHEDPEVKFNW YVDGVEVHNAKTKPREEQYNSTYRVVSVLTVLHQDW LNGKEYKCKVSNKALPAPIEKTISKAKGQPREPQVYTL PPSRDELTKNQVSLTCLVKGFYPSDIAVEWESNGQPEN NYKTTPPVLDSDGSFFLYSKLTVDKSRWQQGNVFSCS VMHEALHNHYTQKSLSLSPGK (SEQ ID NO: 81) |
| A14 | EVQLQESGGGLVQP GGSLRLSCAASGSIF SVAGMGWHRQAPG KQREWVATITRGGR ITYADSVKGRFTISR HIAENTVYLQMMSL NPEDTAVYYCTTSG YWGQGTQVTVSS (SEQ ID NO: 13) | EVQLQESGGGLVQPGGSLRLSCAASGSIFSVAGMGWH RQAPGKQREWVATITRGGRITYADSVKGRFTISRHIAE NTVYLQMMSLNPEDTAVYYCTTSGYWGQGTQVTVSS EPKSSDKTHTCPPCPAPELLGGPSVFLFPPKPKDTLMIS RTPEVTCVVVDVSHEDPEVKFNWYVDGVEVHNAKTK PREEQYNSTYRVVSVLTVLHQDWLNGKEYKCKVSNK ALPAPIEKTISKAKGQPREPQVYTLPPSRDELTKNQVSL TCLVKGFYPSDIAVEWESNGQPENNYKTTPPVLDSDGS FFLYSKLTVDKSRWQQGNVFSCSVMHEALHNHYTQK SLSLSPGK (SEQ ID NO: 82) |

**Table 3. CDR sequences of VHH defined according to the AbM numbering system**

| Clone ID | CDR1 | CDR2 | CDR3 |
|---|---|---|---|
| C23 | GFIISTYSMS (SEQ ID NO: 23) | DINSGGGSTY (SEQ ID NO: 24) | RTQRTYYSGNYYGGGMDY (SEQ ID NO: 25) |
| A13 | GSTSSNHAVG (SEQ ID NO: 26) | SIDSGGSTY (SEQ ID NO: 27) | RTWRGDLY (SEQ ID NO: 28) |
| A2 | GRTFSSNNMG (SEQ ID NO: 29) | ATKWSSGVLY (SEQ ID NO: 30) | MSRVAAMSRLVGDYDY (SEQ ID NO: 31) |
| A3 | GPGFSRYSVG (SEQ ID NO: 32) | TIMWLGDTTY (SEQ ID NO: 33) | KTSLRQYTNRDEYDY (SEQ ID NO: 34) |
| A77 | GFTFSSYDMS (SEQ ID NO: 35) | FITSAGSSTN (SEQ ID NO: 36) | EGAYYSGNYYYTMHDY (SEQ ID NO: 37) |
| A83 | GNIFSINAMG (SEQ ID NO: 38) | ALSSGGRTY (SEQ ID NO: 39) | VNSGSYYSDDY (SEQ ID NO: 40) |
| B102 | GNIFSIAGMG (SEQ ID NO: 41) | TITRGGRIT (SEQ ID NO: 42) | SGY (SEQ ID NO: 43) |
| B91 | GFTFSSYAMS (SEQ ID NO: 44) | DISSAGDSTN (SEQ ID NO: 45) | EGAYYNGNYYYTMHDY (SEQ ID NO: 46) |
| C184 | GFTFSSYGLS (SEQ ID NO: 47) | DINSGGGSTL (SEQ ID NO: 48) | VVQGAGTWYWDRYLEV (SEQ ID NO: 49) |
| C357 | IDTFTLNAIS (SEQ ID NO: 50) | RSWRSGDTN (SEQ ID NO: 51) | IRGADDF (SEQ ID NO: 52) |
| C59 | GFTFSSYDMS (SEQ ID NO: 53) | FITSAGGSTN (SEQ ID NO: 54) | EGAYYNGNYYYTMHDY (SEQ ID NO: 55) |
| D16 | IPDFSRYSVG (SEQ ID NO: 56) | TIMWLGDTTY (SEQ ID NO: 57) | KTSLRQYTNPNEYDY (SEQ ID NO: 58) |
| A14 | GSIFSVAGMG (SEQ ID NO: 59) | TITRGGRIT (SEQ ID NO: 60) | SGY (SEQ ID NO: 61) |

### Example 3 Construction, Expression, and Purification of Antibodies

The 13 nanobodies obtained in Example 2 were constructed as heavy chain antibodies of human IgG1 subtype, i.e., VHH-Fc.

### 3.1 Plasmid Construction

The DNA fragment encoding VHH was constructed into the eukaryotic expression vector plasmid pcDNA3.4-IgG1, which was obtained by ligating the human IgG1 Fc fragment (SEQ ID NO: 69) into the backbone pcDNA3.4 (purchased from Invitrogen, A14697), thereby obtaining a protein expression plasmid containing the complete VHH-Fc full-length gene.

### SEQ ID NO: 69

EPKSSDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSPGK

### 3.2 Expression and Purification of Antibodies

The constructed plasmid was transfected into Expi CHO cells (Thermo Fisher, A29133) for transient expression. 7 days after transfection, the culture supernatant of cells expressing the protein of interest was high-speed centrifuged at 15000 g for 10 min. The resulting supernatant was subjected to affinity purification with Protein A (purchased from Cytiva (GE Life)). Finally, the obtained protein was stored in PBS buffer.

### Example 4 Identification of Physicochemical Properties of Antibodies

### 4.1 Identification of VHH-Fc Antibody by SDS-PAGE

Samples preparation: 1 µg of VHH-Fc antibody was added to 4× LDS loading buffer (containing iodoacetamide at a final concentration of 40 mM), heated in a dry bath at 75°C for 10 min, cooled to room temperature, and then centrifuged at 12,000 rpm for 5 min. The supernatant was used as a sample for non-reducing SDS-PAGE. 2 µg of VHH-Fc antibody was added to 4× LDS loading buffer (containing DTT at a final concentration of 5 mM), heated in a dry bath at 100°C for 10 min, cooled to room temperature, centrifuged at 12000 rpm for 5 min. The supernatant was taken as a sample for reducing SDS-PAGE. For the quality control sample IPI (Ipilimumab), the non-reducing band has a molecular weight of about 150 kDa with a purity of more than 90%, and the reducing band has a heavy chain molecular weight of about 55 kDa and a light chain molecular weight of about 25 kDa with a purity of heavy chain plus light chain of more than 90%. The samples were added to the pre-prepared gel for gel electrophoresis and stained with Coomassie Brilliant Blue, and then scanned with an EPSON V550 color scanner after destaining. The purities of reducing and non-reducing bands were calculated using Image J according to the peak area normalization method.

The results are shown in Table 3: The band of VHH-Fc antibody in the non-reducing gel showed a molecular weight of about 80kD, and the band in the reducing gel showed a molecular weight of about 40 kDa, which were in line with the expected size. Except for C184-Fc, the purities of all other antibodies were greater than 90%.

### 4.2 Purity Identification of Monomer of VHH-Fc Antibody by SEC-HPLC

Materials preparation: 1. Mobile phase preparation: 150 mmol/L PB + NaCl, and pH adjusted to 6.0; 2. Sample processing: the sample diluted to a concentration of 0.5 mg/mL; 3. Agilent HPLC 1100 chromatographic column (XBridge BEH SEC 3.5 µm, 7.8 mm ×300 mm, Waters), flow rate set to 0.8 mL/min, injection volume of 20 µ L, and detector wavelength of 280 nm. The percentages of high-molecular-weight polymer, monomer, and low-molecular-weight polymer in the sample were calculated according to the area normalization method. The results are shown in Table 4. Except for C184-Fc, the purities of VHH-Fc antibody monomers were all greater than 95%.

**Table 4. Detection results of physicochemical properties of VHH-Fc antibodies**

| VHH-Fc antibody | SDS-PAGE Purity | SEC Purity % |
|---|---|---|
| C23-Fc | 94.6 | 96.75 |
| A13-Fc | >95.0 | 96.39 |
| A2-Fc | >95.0 | 95.21 |
| A3-Fc | >95.0 | 96.55 |
| A77-Fc | >95.0 | 95.51 |
| A83-Fc | 91.3 | 96.38 |
| B102-Fc | >95.0 | 95.03 |
| B91-Fc | >95.0 | 95.91 |
| C184-Fc | 82.4 | Abnormal peak shape |
| C357-Fc | >95.0 | 96.43 |
| C59-Fc | >95.0 | 99.59 |
| D16-Fc | >95.0 | 96.36 |
| A14-Fc | >95.0 | 95.91 |

### Example 5 Affinity (ELISA-Binding) Detection of VHH-Fc Antibody

The antigens Human B7-H3-His, Mouse B7-H3-Fc, and Cyno B7-H3-His were diluted with 1× PBS to a concentration of 2 µg/mL and added to a 96-well plate at 30 µL/well for coating overnight at 4°C. The next day, the 96-well plate was washed three times with PBST and then blocked with 5% skim milk for 2 h. After washing 3 times with PBST, the plate was added with the test antibody diluted in 1% PBSM and incubated for 1 h, with Ipilimumab (IPI) as a negative control and DX008-BMG-MGC (self-made sample, the amino acid sequence of the heavy chain set forth in SEQ ID NO: 21 and the amino acid sequence of the light chain set forth in SEQ ID NO: 22) as a positive control. After washing 3 times with PBST, secondary antibodies were added to the plate, with Anti-human-Fc-HRP (abcam; ab97225) for human B7-H3-His and Cyno B7-H3-His, Anti-VHH1+VHH2-HRP (Genescript, A01861-200, CP0001) for Mouse B7-H3-Fc, as well as Anti-human κ+λ-HRP (Millipore; AP502P; AP506P) for Ipilimumab and DX008-BMG-MGC, and the plate was incubated for 1h. After completion of incubation, the plate was washed six times with PBST, and TMB was added for color development. Based on the results of color development, 2M HCl was added to stop the reaction, and the plate was read at OD450 by a microplate reader (Molecular Devices, SpecterMax 190).

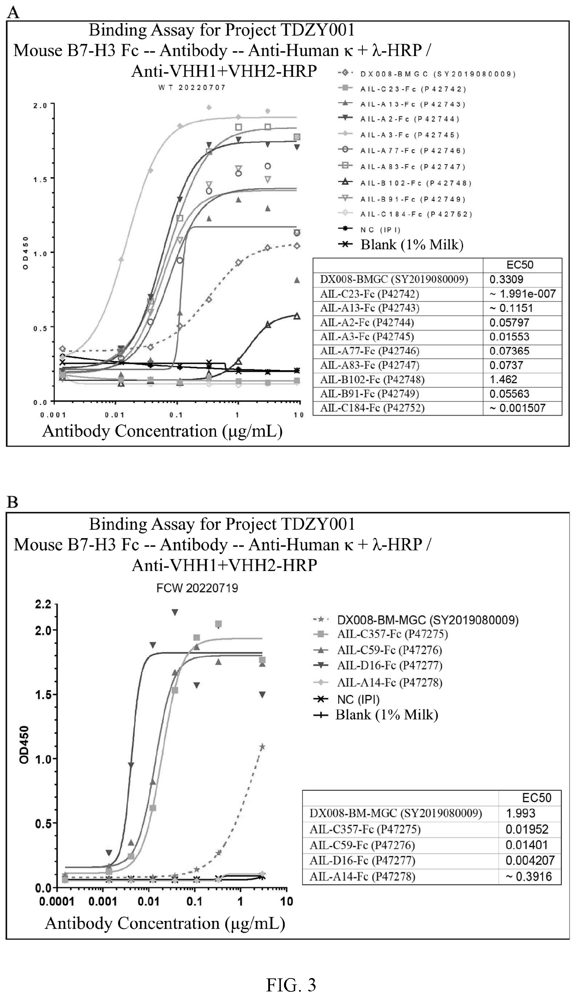

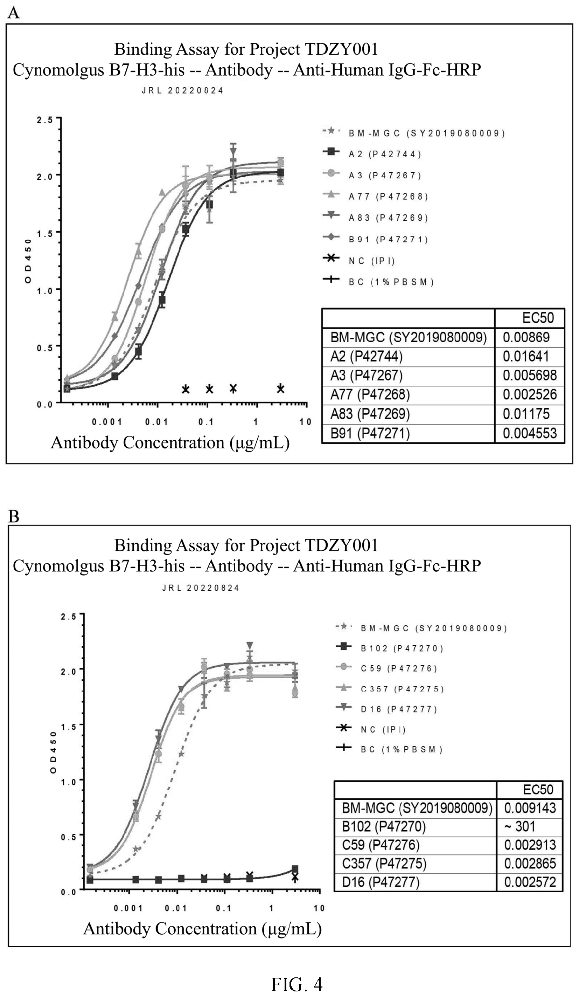

The results are shown in FIG. 2, FIG. 3, and FIG. 4. The results showed that, except for C184, C23, and A13, all other antibodies showed high affinity activity for Human B7-H3-His antigen. A13, A2, A3, A77, A83, B91, C357, C59 and D16 exhibited human-mouse cross-activity. Nine antibodies were selected for the determination of cross-reactivity between human and monkey, and the results showed that A2, A3, A77, A83, B91, C59, C357, and D16 exhibited a human-monkey cross-reactivity between human and monkey.
DX008-BM-MGC Positive Control Antibody VH (SEQ ID NO: 21) 
EVQLVESGGGLVKPGGSLRLSCAASGFTFSSYGMSWVRQAPGKGLEWVATINS
GGSNTYYPDSLKGRFTISRDNAKNSLYLQMNSLRAEDTAVYYCARHDGGAMDYWGQG
TTVTVSS
DX008-BM-MGC Positive Control Antibody VL (SEQ ID NO: 22)

DIQMTQSPSSLSASVGDRVTITCRASESIYSYLAWYQQKPGKAPKLLVYNTKTLP

EGVPSRFSGSGSGTDFTLTISSLQPEDFATYYCQHHYGTPPWTFGQGTRLEIK

### Example 6 Kinetic Determination of Affinity of VHH-Fc Antibody

The affinity kinetics of VHH antibodies for antigens Human B7-H3-His and Mouse B7-H3-His were detected based on the GATOR-based Kinetics experimental mode of GATOR. DX008-BM-MGC was used as a positive control. The antibody to be tested was diluted to 30 nM in a buffer containing PBS (10 mM, pH 7.4), 0.02% Tween 20, and 0.2% BSA, and then subjected to a preset procedure with an association time of 120 s and a dissociation time of 120 s. The antigen was serially diluted 2-fold from 2400 nM to 4.69 nM. Finally, KD (affinity kinetic constant), Kon (association constant), and Koff (dissociation constant) were fitted from the association and dissociation data of different concentrations of antigens. Kon is also written as Ka, and Koff is also written as Kd.

The detection results are shown in Tables 5 and 6. The results showed that the affinity of antibodies A2, A77, B91, and C59 was higher than that of the control antibody, and the affinity of antibodies A3, A83, B102, C357, and D16 was similar or slightly lower than that of the control antibody, among which A2, A77, B91, C357, and C59 also had affinity for murine B7-H3 antigen.

**Table 5. Kinetic detection results of the affinity of the VHH-Fc antibody to antigen Human B7-H3-His**

| Sample Name | Antigen Human B7-H3-His | | | |
|---|---|---|---|---|
| | KD (M) | Ka(1/Ms) | Kd(1/s) | Rmax(nm) |
| DX008-BM-MGC | 1.19E-08 | 3.16E+05 | 3.75E-03 | 0.998 |
| AIL-A2-Fc | 3.68E-09 | 1.32E+05 | 4.86E-04 | 0.996 |
| AIL-A3-Fc | 1.40E-07 | 1.75E+04 | 2.45E-03 | 0.969 |
| AIL-A77-Fc | 4.06E-09 | 6.76E+04 | 2.74E-04 | 0.996 |
| AIL-A83-Fc | 2.98E-07 | 3.09E+03 | 9.20E-04 | 0.974 |
| AIL-B102-Fc | 5.27E-08 | 2.62E+05 | 1.38E-02 | 0.956 |
| AIL-B91-Fc | 2.39E-09 | 6.05E+04 | 1.44E-04 | 0.996 |
| AIL-C357-Fc | 1.11E-07 | 2.87E+05 | 3.18E-02 | 0.973 |
| AIL-C59-Fc | 3.17E-09 | 7.77E+04 | 2.46E-04 | 0.995 |
| AIL-D16-Fc | 4.71E-07 | 1.43E+04 | 6.74E-03 | 0.961 |

**Table 6. Kinetic determination results of the affinity of the VHH-Fc antibody to antigen Mouse B7-H3-His**

| Sample Name | Antigen Mouse B7-H3 Protein | | | |
|---|---|---|---|---|
| | KD(M) | Ka(1/Ms) | Kd(1/s) | Rmax(nm) |
| DX008-BM-MGC | N/A | N/A | N/A | N/A |
| AIL-A2-Fc | 1.14E-08 | 7.20E+04 | 8.23E-04 | 0.995 |
| AIL-A3-Fc | N/A | N/A | N/A | N/A |
| AIL-A77-Fc | 1.17E-08 | 5.88E+04 | 6.88E-04 | 0.995 |
| AIL-A83-Fc | N/A | N/A | N/A | N/A |
| AIL-B102-Fc | N/A | N/A | N/A | N/A |
| AIL-B91-Fc | 1.43E-08 | 4.92E+04 | 7.05E-04 | 0.996 |
| AIL-C357-Fc | 6.44E-08 | 5.48E+04 | 3.53E-03 | 0.998 |
| AIL-C59-Fc | 5.23E-09 | 5.42E+04 | 2.84E-04 | 0.996 |
| AIL-D16-Fc | N/A | N/A | N/A | N/A |

### Example 7 Humanization of Nanobodies

In order to reduce the potential immunogenicity caused by camel-derived nanobodies, the framework region of the nanobodies were subjected to humanizing mutations and back mutations to obtain a heavy chain antibody with a high degree of humanization while maintaining the affinity of the humanized antibody for the antigen.

### 7.1 Humanization Process of Nanobodies

The VHH antibody A2 was selected for humanization. The antibody sequence was aligned with the human antibody Germline gene database to find a human Germline with high sequence homology with the VHH. After defining the CDRs and the framework regions, varying degrees of the humanized sequences were designed based on the differing sites in the framework region. Seven humanized antibodies, VHH21 to VHH27, were obtained after the humanization of the A2 sequence, the amino acid sequences of which are shown in Table 7.

Expression vectors for humanized antibodies VHH21 to VHH27 were constructed as described in Example 3.1. These humanized VHH-Fc antibodies each comprised the VHH amino acid sequence as described above, and the Fc fragment of human IgG1 (SEQ ID NO: 69). Expression and purification of the humanized VHH-Fc antibodies were performed as described in Example 3.2. The sequences of the humanized VHH-Fc antibodies are shown in Table 7.

**Table 7. Sequences of humanized VHH and humanized VHH-Fc of A2 VHH**

| | Sequence of Humanized VHH | Full-length sequence of humanized VHH-Fc antibody |
|---|---|---|
| VHH21 | QVQLVESGGGLVQAG GSLRLTCAASGRTFSS NNMGWFRQAPGKERE FVTATKWSSGVLYYA DSADSVKGRFTNSRDV ARNTVYLHMTNLRAE DTAVYYCAAMSRVAA MSRLVGDYDYWGQG TLVTVSS (SEQ ID NO: 14) | QVQLVESGGGLVQAGGSLRLTCAASGRTFSSN NMGWFRQAPGKEREFVTATKWSSGVLYYADS ADSVKGRFTNSRDVARNTVYLHMTNLRAEDT AVYYCAAMSRVAAMSRLVGDYDYWGQGTLV TVSSEPKSSDKTHTCPPCPAPELLGGPSVFLFPP KPKDTLMISRTPEVTCVVVDVSHEDPEVKFNW YVDGVEVHNAKTKPREEQYNSTYRVVSVLTV LHQDWLNGKEYKCKVSNKALPAPIEKTISKAK GQPREPQVYTLPPSRDELTKNQVSLTCLVKGFY PSDIAVEWESNGQPENNYKTTPPVLDSDGSFFL YSKLTVDKSRWQQGNVFSCSVMHEALHNHYT QKSLSLSPGK (SEQ ID NO: 62) |
| VHH22 | EVQLVESGGGLVQAG GSLRLTCAASGRTFSS NNMGWFRQAPGKERE FVTATKWSSGVLYYA DSADSVKGRFTISRDN ARNTVYLHMTNLKPE DTAVYYCAAMSRVAA MSRLVGDYDYWGQG TLVTVSS (SEQ ID NO: 15) | EVQLVESGGGLVQAGGSLRLTCAASGRTFSSN NMGWFRQAPGKEREFVTATKWSSGVLYYADS ADSVKGRFTISRDNARNTVYLHMTNLKPEDTA VYYCAAMSRVAAMSRLVGDYDYWGQGTLVT VSSEPKSSDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPSRDELTKNQVSLTCLVKGFYP SDIAVEWESNGQPENNYKTTPPVLDSDGSFFLY SKLTVDKSRWQQGNVFSCSVMHEALHNHYTQ KSLSLSPGK (SEQ ID NO: 63) |
| VHH23 | QVQLVESGGGLVQAG GSLRLTCAASGRTFSS NNMGWFRQAPGKERE FVTATKWSSGVLYYA DSADSVKGRFTNSRDN AKNTLYLHMTNLKPE DTAVYYCAAMSRVAA MSRLVGDYDYWGQG TLVTVSS (SEQ ID NO: 16) | QVQLVESGGGLVQAGGSLRLTCAASGRTFSSN NMGWFRQAPGKEREFVTATKWSSGVLYYADS ADSVKGRFTNSRDNAKNTLYLHMTNLKPEDT AVYYCAAMSRVAAMSRLVGDYDYWGQGTLV TVSSEPKSSDKTHTCPPCPAPELLGGPSVFLFPP KPKDTLMISRTPEVTCVVVDVSHEDPEVKFNW YVDGVEVHNAKTKPREEQYNSTYRVVSVLTV LHQDWLNGKEYKCKVSNKALPAPIEKTISKAK GQPREPQVYTLPPSRDELTKNQVSLTCLVKGFY PSDIAVEWESNGQPENNYKTTPPVLDSDGSFFL YSKLTVDKSRWQQGNVFSCSVMHEALHNHYT QKSLSLSPGK (SEQ ID NO: 64) |
| VHH2 4 | EVQLVESGGGLVQAG GSLRLSCAASGRTFSS NNMGWFRQAPGKERE FVTATKWSSGVLYYA DSADSVKGRFTISRDV AKNTVYLHMTNLKPE DTAVYYCAAMSRVAA MSRLVGDYDYWGQG TLVTVSS (SEQ ID NO: 17) | EVQLVESGGGLVQAGGSLRLSCAASGRTFSSN NMGWFRQAPGKEREFVTATKWSSGVLYYADS ADSVKGRFTISRDVAKNTVYLHMTNLKPEDTA VYYCAAMSRVAAMSRLVGDYDYWGQGTLVT VSSEPKSSDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPSRDELTKNQVSLTCLVKGFYP SDIAVEWESNGQPENNYKTTPPVLDSDGSFFLY SKLTVDKSRWQQGNVFSCSVMHEALHNHYTQ KSLSLSPGK (SEQ ID NO: 65) |
| VHH2 5 | QVQLVESGGGLVQAG GSLRLSCAASGRTFSS NNMGWFRQAPGKERE FVTATKWSSGVLYYA DSADSVKGRFTNSRDV ARNTVYLHMTNLRAE DTAVYYCAAMSRVAA MSRLVGDYDYWGQG TLVTVSS (SEQ ID NO: 18) | QVQLVESGGGLVQAGGSLRLSCAASGRTFSSN NMGWFRQAPGKEREFVTATKWSSGVLYYADS ADSVKGRFTNSRDVARNTVYLHMTNLRAEDT AVYYCAAMSRVAAMSRLVGDYDYWGQGTLV TVSSEPKSSDKTHTCPPCPAPELLGGPSVFLFPP KPKDTLMISRTPEVTCVVVDVSHEDPEVKFNW YVDGVEVHNAKTKPREEQYNSTYRVVSVLTV LHQDWLNGKEYKCKVSNKALPAPIEKTISKAK GQPREPQVYTLPPSRDELTKNQVSLTCLVKGFY PSDIAVEWESNGQPENNYKTTPPVLDSDGSFFL YSKLTVDKSRWQQGNVFSCSVMHEALHNHYT QKSLSLSPGK (SEQ ID NO: 66) |
| VHH2 6 | EVQLVESGGGLVQAG GSLRLSCAASGRTFSS NNMGWFRQAPGKERE FVTATKWSSGVLYYA DSADSVKGRFTISRDN AKNTVYLHMTNLKPE DTAVYYCAAMSRVAA MSRLVGDYDYWGQG TLVTVSS (SEQ ID NO: 19) | EVQLVESGGGLVQAGGSLRLSCAASGRTFSSN NMGWFRQAPGKEREFVTATKWSSGVLYYADS ADSVKGRFTISRDNAKNTVYLHMTNLKPEDTA VYYCAAMSRVAAMSRLVGDYDYWGQGTLVT VSSEPKSSDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPSRDELTKNQVSLTCLVKGFYP SDIAVEWESNGQPENNYKTTPPVLDSDGSFFLY SKLTVDKSRWQQGNVFSCSVMHEALHNHYTQ KSLSLSPGK (SEQ ID NO: 67) |
| VHH2 7 | EVQLVESGGGLVQAG GSLRLSCAASGRTFSS NNMGWFRQAPGKERE FVTATKWSSGVLYYA DSADSVKGRFTISRDN | EVQLVESGGGLVQAGGSLRLSCAASGRTFSSN NMGWFRQAPGKEREFVTATKWSSGVLYYADS ADSVKGRFTISRDNAKNTVYLHMTNLRAEDTA VYYCAAMSRVAAMSRLVGDYDYWGQGTLVT VSSEPKSSDKTHTCPPCPAPELLGGPSVFLFPPK |
| | AKNTVYLHMTNLRAE DTAVYYCAAMSRVAA MSRLVGDYDYWGQG TLVTVSS (SEQ ID NO: 20) | PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPSRDELTKNQVSLTCLVKGFYP SDIAVEWESNGQPENNYKTTPPVLDSDGSFFLY SKLTVDKSRWQQGNVFSCSVMHEALHNHYTQ KSLSLSPGK (SEQ ID NO: 68) |

**Table 8. CDR sequences of humanized VHH of A2 VHH**

| Clone ID | CDR1 | CDR2 | CDR3 |
|---|---|---|---|
| A2 | GRTFSSNNMG (SEQ ID NO: 29) | ATKWSSGVLY (SEQ ID NO: 30) | MSRVAAMSRLVGDYDY (SEQ ID NO: 31) |
| VHH21 | GRTFSSNNMG (SEQ ID NO: 29) | ATKWSSGVLY (SEQ ID NO: 30) | MSRVAAMSRLVGDYDY (SEQ ID NO: 31) |
| VHH22 | GRTFSSNNMG (SEQ ID NO: 29) | ATKWSSGVLY (SEQ ID NO: 30) | MSRVAAMSRLVGDYDY (SEQ ID NO: 31) |
| VHH23 | GRTFSSNNMG (SEQ ID NO: 29) | ATKWSSGVLY (SEQ ID NO: 30) | MSRVAAMSRLVGDYDY (SEQ ID NO: 31) |
| VHH24 | GRTFSSNNMG (SEQ ID NO: 29) | ATKWSSGVLY (SEQ ID NO: 30) | MSRVAAMSRLVGDYDY (SEQ ID NO: 31) |
| VHH25 | GRTFSSNNMG (SEQ ID NO: 29) | ATKWSSGVLY (SEQ ID NO: 30) | MSRVAAMSRLVGDYDY (SEQ ID NO: 31) |
| VHH26 | GRTFSSNNMG (SEQ ID NO: 29) | ATKWSSGVLY (SEQ ID NO: 30) | MSRVAAMSRLVGDYDY (SEQ ID NO: 31) |
| VHH27 | GRTFSSNNMG (SEQ ID NO: 29) | ATKWSSGVLY (SEQ ID NO: 30) | MSRVAAMSRLVGDYDY (SEQ ID NO: 31) |

### 7.2 Affinity Activity Assay of Humanized Antibodies (ELISA-Binding Assay)

The affinity of humanized VHH-Fc antibodies (VHH21 to VHH27) for antigens Human B7-H3-His, Mouse B7-H3-His, and Cyno B7-H3-His was determined by ELISA and compared with the parent antibody A2-VHH-Fc.

The specific method was as follows. The antigens Human B7-H3-His, Mouse B7-H3-His, or Cyno B7-H3-His were diluted with 1× PBS to a concentration of 2 µg/mL, and added to a 96-well plate at 30 µL/well for coating overnight at 4°C. The next day, the plate was washed three times with PBST and blocked with 5% PBS-Milk for 2 hours. After washing, the plate was added with serially-diluted VHH-Fc antibody to be tested (VHH21 to VHH27; Ipilimumab (IPI) as a negative control and DX008-BMG-MGC as a positive control) and incubated for 1 h. After washing three times with PBST, the plate was added with the secondary antibody Anti-human-IgG-Fc-HRP (abcam, ab97225) and incubated at room temperature for 60 min. The plate was washed six times with PBST, and TMB was added for color development. Based on the color development results, 2M HCl was added to stop the reaction, and the plate was detected at OD450.

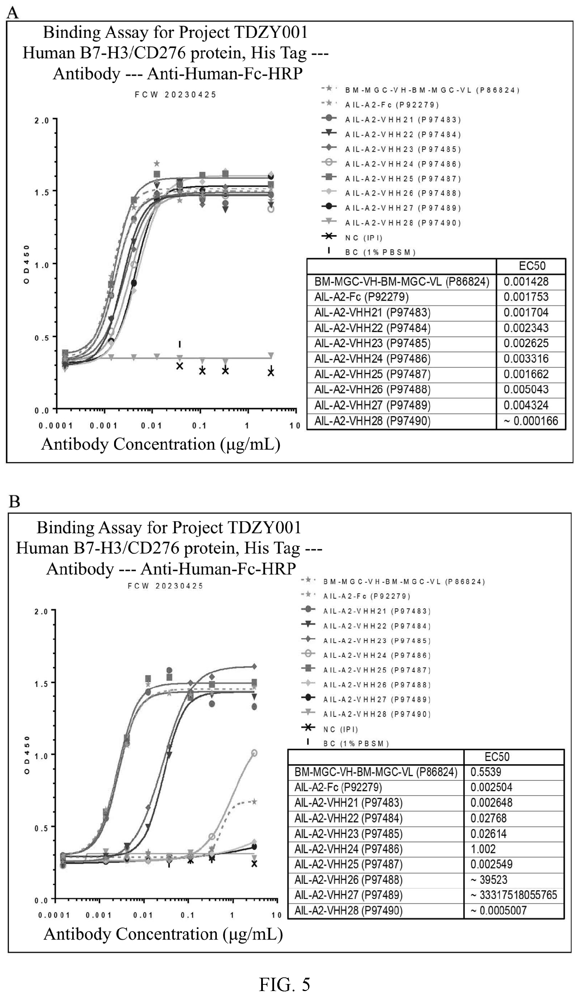

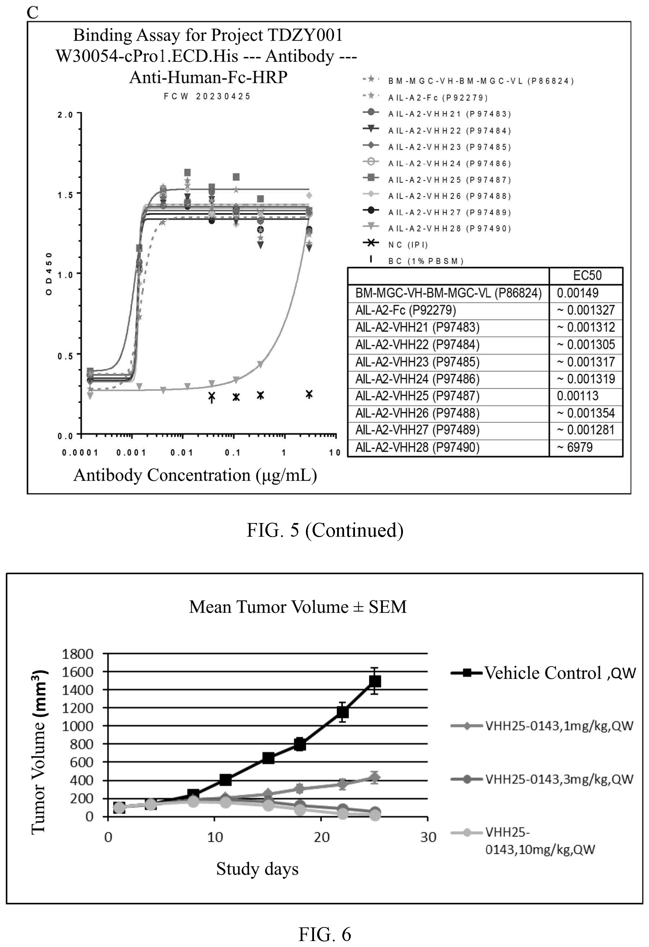

The results are shown in FIG. 5. The results showed that VHH21-Fc to VHH27-Fc all maintained the affinity for human and cynomolgus monkey B7-H3 antigens, and VHH21-Fc, VHH22-Fc, VHH23-Fc, and VHH25-Fc retained the binding ability of the parent to mouse antigen.

### Example 8 Affinity Kinetics Assessment of Humanized Antibodies

The affinity kinetics of the humanized VHH-Fc antibodies for the antigens Human B7-H3-His, Mouse B7-H3-His, and Cyno B7-H3-His were detected based on the Kientics experimental mode of GATOR, with the parent antibody A2 VHH-Fc as a positive control. The detection was performed according to Example 6.

The detection results are shown in Table 9. The results showed that the KD value of VHH21 and VHH25 were comparable to that of the parent, and the KD value of other molecules was greater than that of the parent A2VHH-Fc antibody.

**Table 9. Kinetic detection results of the affinity of humanized VHH-Fc antibodies to the antigens Human B7-H3-His, Mouse B7-H3-His, and Cyno B7-H3-His**

| Protein Name | Human B7-H3-His | | | Mouse B7-H3-His | | | Cyno B7-H3-His | | |
|---|---|---|---|---|---|---|---|---|---|
| | KD (M) | ka(1/Ms) | kd(1/s) | KD (M) | ka(1/Ms) | kd(1/s) | KD (M) | ka(1/Ms) | kd(1/s) |
| AIL-A2-Fc | 3.96E-09 | 6.55E+04 | 2.59E-04 | 1.13E-08 | 8.91E+04 | 1.01E-03 | 2.17E-10 | 1.37E+05 | 2.97E-05 |
| VHH21-Fc | 3.81E-09 | 6.52E+04 | 2.48E-04 | 1.25E-08 | 8.22E+04 | 1.03E-03 | NA | 1.44E+05 | NA |
| VHH22-Fc | 1.36E-07 | 8.79E+04 | 1.20E-02 | 2.86E-07 | 1.33E+05 | 3.81E-02 | 1.94E-10 | 1.18E+05 | 2.29E-05 |
| VHH23-Fc | 2.05E-08 | 6.01E+04 | 1.23E-03 | 1.19E-07 | 1.34E+05 | 1.59E-02 | NA | 1.33E+05 | NA |
| VHH24-Fc | 1.28E-07 | 6.88E+04 | 8.84E-03 | 1.46E-06 | 7.09E+04 | 1.04E-01 | NA | 1.08E+05 | NA |
| VHH25-Fc | 2.92E-09 | 6.62E+04 | 1.93E-04 | 8.96E-09 | 8.21E+04 | 7.36E-04 | NA | 1.49E+05 | NA |
| VHH26-Fc | 2.29E-07 | 7.36E+04 | 1.68E-02 | 6.54E-08 | 3.12E+05 | 2.04E-02 | 1.20E-09 | 1.26E+05 | 1.52E-04 |
| VHH27-Fc | 1.57E-07 | 1.01E+05 | 1.59E-02 | 4.60E-08 | 2.91E+05 | 1.34E-02 | 2.33E-10 | 1.12E+05 | 2.61E-05 |

| | | | | | | | | | |
|---|---|---|---|---|---|---|---|---|---|
| **Note: N/A represents no association at 1200 nM, and NA represents not dissociation.** | | | | | | | | | |

### Example 9 In Vivo Pharmacodynamic Experiment of ADC of Humanized Candidate Molecule

A VHH25-0143 ADC molecule, with a DAR value of about 4, was prepared by chemically coupling topoisomerase toxin 0143 (available from MCE, Cat#: HY-114233), formed by linking a DNA topoisomerase I inhibitor DX8951 and a protease cleavable linker MC-GGFG, to the humanized antibody molecule VHH25-Fc of the present application. The structure of the topoisomerase Toxin 0143 is as follows:

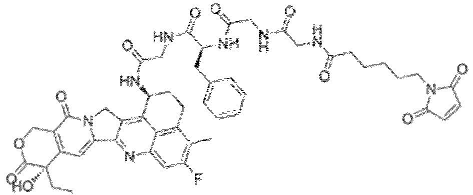

Calu-6 cells were subcutaneously inoculated into the right dorsal side of Balb/c nude mice to establish a subcutaneous xenograft mouse model of human lung cancer Calu-6 cell line. When the tumors grew to an average volume of 100 mm³, mice were randomly divided into four groups based on tumor size and body weight of mice: Vehicle Control group, VHH25-0143 ADC low dose group (1 mg/kg), VHH25-0143 ADC medium dose group (3 mg/kg) and VHH25-0143 ADC high dose group (10 mg/kg), with 5 mice per group. The day of grouping was recorded as Day 1, and dosing was started on the day of grouping, once a week (QW), for a total of 3 doses. Tumor size was measured twice a week after dosing, and the results are shown in FIG. 6.

As shown in FIG. 6, the VHH25-0143 ADC molecule inhibited tumor growth at all three doses, indicating that it has potent anti-tumor activity *in vivo.*

## Claims

1. A B7-H3 binding protein comprising an immunoglobulin single variable domain, wherein the immunoglobulin single variable domain comprises CDR1, CDR2, and CDR3 comprised in a VHH set forth in any one of SEQ ID NOs: 1-13.

2. The B7-H3 binding protein of claim 1, wherein the CDR1, CDR2, and CDR3 are defined according to Kabat, AbM, Chothia, or IMGT.

3. The B7-H3 binding protein of claim 2, wherein the CDR1, CDR2, and CDR3 are defined according to AbM, and:
(1) the CDR1 comprises an amino acid sequence having at least 85%, at least 90%, at least 95%, or 100% identity to the amino acid sequence set forth in SEQ ID NO: 29, the CDR2 comprises an amino acid sequence having at least 85%, at least 90%, at least 95%, or 100% identity to the amino acid sequence set forth in SEQ ID NO: 30, and the CDR3 comprises an amino acid sequence having at least 85%, at least 90%, at least 95%, or 100% identity to the amino acid sequence set forth in SEQ ID NO: 31;
(2) the CDR1 comprises an amino acid sequence having at least 85%, at least 90%, at least 95%, or 100% identity to the amino acid sequence set forth in SEQ ID NO: 23, the CDR2 comprises an amino acid sequence having at least 85%, at least 90%, at least 95%, or 100% identity to the amino acid sequence set forth in SEQ ID NO: 24, and the CDR3 comprises an amino acid sequence having at least 85%, at least 90%, at least 95%, or 100% identity to the amino acid sequence set forth in SEQ ID NO: 25;
(3) the CDR1 comprises an amino acid sequence having at least 85%, at least 90%, at least 95%, or 100% identity to the amino acid sequence set forth in SEQ ID NO: 26, the CDR2 comprises an amino acid sequence having at least 85%, at least 90%, at least 95%, or 100% identity to the amino acid sequence set forth in SEQ ID NO: 27, and the CDR3 comprises an amino acid sequence having at least 85%, at least 90%, at least 95%, or 100% identity to the amino acid sequence set forth in SEQ ID NO: 28;
(4) the CDR1 comprises an amino acid sequence having at least 85%, at least 90%, at least 95%, or 100% identity to the amino acid sequence set forth in SEQ ID NO: 32, the CDR2 comprises an amino acid sequence having at least 85%, at least 90%, at least 95%, or 100% identity to the amino acid sequence set forth in SEQ ID NO: 33, and the CDR3 comprises an amino acid sequence having at least 85%, at least 90%, at least 95%, or 100% identity to the amino acid sequence set forth in SEQ ID NO: 34;
(5) the CDR1 comprises an amino acid sequence having at least 85%, at least 90%, at least 95%, or 100% identity to the amino acid sequence set forth in SEQ ID NO: 35, the CDR2 comprises an amino acid sequence having at least 85%, at least 90%, at least 95%, or 100% identity to the amino acid sequence set forth in SEQ ID NO: 36, and the CDR3 comprises an amino acid sequence having at least 85%, at least 90%, at least 95%, or 100% identity to the amino acid sequence set forth in SEQ ID NO: 37;
(6) the CDR1 comprises an amino acid sequence having at least 85%, at least 90%, at least 95%, or 100% identity to the amino acid sequence set forth in SEQ ID NO: 38, the CDR2 comprises an amino acid sequence having at least 85%, at least 90%, at least 95%, or 100% identity to the amino acid sequence set forth in SEQ ID NO: 39, and the CDR3 comprises an amino acid sequence having at least 85%, at least 90%, at least 95%, or 100% identity to the amino acid sequence set forth in SEQ ID NO: 40;
(7) the CDR1 comprises an amino acid sequence having at least 85%, at least 90%, at least 95%, or 100% identity to the amino acid sequence set forth in SEQ ID NO: 41, the CDR2 comprises an amino acid sequence having at least 85%, at least 90%, at least 95%, or 100% identity to the amino acid sequence set forth in SEQ ID NO: 42, and the CDR3 comprises an amino acid sequence having at least 85%, at least 90%, at least 95%, or 100% identity to the amino acid sequence set forth in SEQ ID NO: 43;
(8) the CDR1 comprises an amino acid sequence having at least 85%, at least 90%, at least 95%, or 100% identity to the amino acid sequence set forth in SEQ ID NO: 44, the CDR2 comprises an amino acid sequence having at least 85%, at least 90%, at least 95%, or 100% identity to the amino acid sequence set forth in SEQ ID NO: 45, and the CDR3 comprises an amino acid sequence having at least 85%, at least 90%, at least 95%, or 100% identity to the amino acid sequence set forth in SEQ ID NO: 46;
(9) the CDR1 comprises an amino acid sequence having at least 85%, at least 90%, at least 95%, or 100% identity to the amino acid sequence set forth in SEQ ID NO: 47, the CDR2 comprises an amino acid sequence having at least 85%, at least 90%, at least 95%, or 100% identity to the amino acid sequence set forth in SEQ ID NO: 48, and the CDR3 comprises an amino acid sequence having at least 85%, at least 90%, at least 95%, or 100% identity to the amino acid sequence set forth in SEQ ID NO: 49;
(10) the CDR1 comprises an amino acid sequence having at least 85%, at least 90%, at least 95%, or 100% identity to the amino acid sequence set forth in SEQ ID NO: 50, the CDR2 comprises an amino acid sequence having at least 85%, at least 90%, at least 95%, or 100% identity to the amino acid sequence set forth in SEQ ID NO: 51, and the CDR3 comprises an amino acid sequence having at least 85%, at least 90%, at least 95%, or 100% identity to the amino acid sequence set forth in SEQ ID NO: 52;
(11) the CDR1 comprises an amino acid sequence having at least 85%, at least 90%, at least 95%, or 100% identity to the amino acid sequence set forth in SEQ ID NO: 53, the CDR2 comprises an amino acid sequence having at least 85%, at least 90%, at least 95%, or 100% identity to the amino acid sequence set forth in SEQ ID NO: 54, and the CDR3 comprises an amino acid sequence having at least 85%, at least 90%, at least 95%, or 100% identity to the amino acid sequence set forth in SEQ ID NO: 55;
(12) the CDR1 comprises an amino acid sequence having at least 85%, at least 90%, at least 95%, or 100% identity to the amino acid sequence set forth in SEQ ID NO: 56, the CDR2 comprises an amino acid sequence having at least 85%, at least 90%, at least 95%, or 100% identity to the amino acid sequence set forth in SEQ ID NO: 57, and the CDR3 comprises an amino acid sequence having at least 85%, at least 90%, at least 95%, or 100% identity to the amino acid sequence set forth in SEQ ID NO: 58; or
(13) the CDR1 comprises an amino acid sequence having at least 85%, at least 90%, at least 95%, or 100% identity to the amino acid sequence set forth in SEQ ID NO: 59, the CDR2 comprises an amino acid sequence having at least 85%, at least 90%, at least 95%, or 100% identity to the amino acid sequence set forth in SEQ ID NO: 60, and the CDR3 comprises an amino acid sequence having at least 85%, at least 90%, at least 95%, or 100% identity to the amino acid sequence set forth in SEQ ID NO: 61.

4. The B7-H3 binding protein of any one of claims 1-3, wherein the immunoglobulin single variable domain comprises an amino acid sequence having at least 85%, at least 90%, at least 95%, or 100% identity to the amino acid sequence set forth in any one of SEQ ID NOs: 1-13, or consists of an amino acid sequence having at least 80%, at least 85%, at least 90%, at least 95%, or 100% identity to the amino acid sequence set forth in any one of SEQ ID NOs: 1-13.

5. The B7-H3 binding protein of any one of claims 1-4, wherein the B7-H3 binding protein is monovalent, bivalent, or multivalent.

6. The B7-H3 binding protein of any one of claims 1-5, wherein the B7-H3 binding protein is monospecific, bispecific, or multispecific.

7. The B7-H3 binding protein of any one of claims 1-3 and 5-6, wherein the immunoglobulin single variable domain comprises a heavy chain framework region, and at least a portion of the heavy chain framework region is derived from at least one of a mouse antibody, a human antibody, a primate antibody, or a mutant thereof.

8. The B7-H3 binding protein of claim 7, wherein at least a portion of the heavy chain framework region is derived from a human antibody, preferably the immunoglobulin single variable domain comprises an amino acid sequence having at least 80%, at least 85%, at least 90%, at least 95% or 100% identity to the amino acid sequence set forth in any one of SEQ ID NOs: 14-20, more preferably the immunoglobulin single variable domain comprises or consists of the amino acid sequence set forth in any one of SEQ ID NOs: 14-20.

9. The B7-H3 binding protein of any one of claims 1-8, wherein the B7-H3 binding protein is a heavy chain antibody, preferably, the heavy chain antibody further comprises a human IgG Fc, more preferably, the heavy chain antibody further comprises a human IgG1 Fc, even more preferably, the human IgG1 Fc comprises an amino acid sequence having at least 80%, at least 85%, at least 90%, at least 95%, or 100% identity to the amino acid sequence set forth in SEQ ID NO: 69, still more preferably, the human IgG1 Fc comprises or consists of the amino acid sequence set forth in SEQ ID NO: 69.

10. The B7-H3 binding protein of any one of claims 1-9, wherein the B7-H3 binding protein is a nanobody.

11. The B7-H3 binding protein of claim 10, wherein the nanobody comprises an amino acid sequence having at least 80%, at least 85%, at least 90%, at least 95% or 100% identity to the amino acid sequence set forth in any one of SEQ ID NOs: 1-13, preferably the nanobody comprises or consists of the amino acid sequence set forth in any one of SEQ ID NOs: 1-13.

12. The B7-H3 binding protein of claim 10, wherein the nanobody comprises an amino acid sequence having at least 80%, at least 85%, at least 90%, at least 95% or 100% identity to the amino acid sequence set forth in any one of SEQ ID NOs: 14-20, preferably the nanobody comprises or consists of the amino acid sequence set forth in any one of SEQ ID NOs: 14-20.

13. A fusion protein comprising the B7-H3 binding protein of any one of claims 1-12.

14. A nucleic acid molecule encoding the B7-H3 binding protein of any one of claims 1-12, or the fusion protein of claim 13, optionally wherein the nucleic acid molecule is DNA.

15. An expression vector comprising the nucleic acid molecule of claim 14, optionally wherein the expression vector is a prokaryotic expression vector or a eukaryotic expression vector.

16. A cell comprising the nucleic acid molecule of claim 14, or the expression vector of claim 15, optionally wherein the cell is a prokaryotic cell or a eukaryotic cell (e.g., a mammalian cell).

17. A conjugate comprising the B7-H3 binding protein of any one of claims 1-12 or the fusion protein of claim 13, conjugated to a therapeutic, diagnostic, or imaging agent; optionally, wherein the therapeutic agent is a small molecule cytotoxic drug.

18. The conjugate of claim 17, wherein the therapeutic agent is a topoisomerase inhibitor, preferably exatecan.

19. The conjugate of claim 17 or 18, wherein the B7-H3 binding protein or the fusion protein is conjugated to the therapeutic, diagnostic, or imaging agent via a linker, preferably the linker is MC-GGFG.

20. A composition comprising the B7-H3 binding protein of any one of claims 1-12, the fusion protein of claim 13, the nucleic acid molecule of claim 14, the expression vector of claim 15, the cell of claim 16, or the conjugate of any one of claims 17-19, optionally the composition is a pharmaceutical composition, and the pharmaceutical composition further comprises a pharmaceutically acceptable carrier, excipient, or diluent.

21. Use of the B7-H3 binding protein of any one of claims 1-12, the fusion protein of claim 13, the nucleic acid molecule of claim 14, the expression vector of claim 15, or the cell of claim 16, or the conjugate of any one of claims 17-19 in the manufacture of a medicament for preventing, treating, or alleviating a B7-H3-associated disease.

22. Use of the B7-H3 binding protein of any one of claims 1-12, the fusion protein of claim 13, the nucleic acid molecule of claim 14, the expression vector of claim 15, or the cell of claim 16, or the conjugate of any one of claims 17-19 in combination with an another agent, in the manufacture of a medicament for preventing, treating, or alleviating a B7-H3-associated disease, preferably the another agent is an immunotherapeutic agent or a chemotherapeutic agent.

23. The use according to claim 21 or 22, wherein the B7-H3-associated disease is a tumor, preferably a solid tumor and/or a hematological tumor, further preferably, lung cancer, breast cancer, prostate cancer, pancreatic cancer, colorectal cancer, melanoma, liver cancer, ovarian cancer, bladder cancer, gastric cancer, esophageal, renal cancer, adrenal tumor, AIDS-associated cancer, alveolar soft part sarcoma, astrocytic tumor, bone cancer, brain and spinal cord cancer, metastatic brain tumor, B-cell cancer, carotid body tumor, chondrosarcoma, chordoma, benign fibrous histiocytoma of the skin, desmoplastic small round cell tumor, ependymoma, Ewing's tumor, extraskeletal myxoid chondrosarcoma, osteogenesis imperfecta, osteofibrous dysplasia, gallbladder cancer or cholangiocarcinoma, gestational trophoblastic disease, germ cell tumor, head and neck cancer, hepatocellular carcinoma, islet cell tumor, Kaposi's sarcoma, leukemia, liposarcoma/malignant lipoma, lymphoma, medulloblastoma, meningioma, multiple endocrine neoplasia, multiple myeloma, myelodysplastic syndrome, neuroblastoma, papillary thyroid carcinoma, parathyroid adenoma, childhood cancer, peripheral nerve sheath tumor, melanocytoma, pituitary tumor, posterior uveal melanoma, renal metastatic cancer, rhabdoid tumor, rhabdomyosarcoma, sarcoma, skin cancer, soft tissue sarcoma, squamous cell cancer, gastric cancer, synovial sarcoma, testicular cancer, thymoma, and thyroid metastatic cancer.

24. The use according to claim 21 or 22, wherein the B7-H3-associated disease is a lung cancer.

25. A kit for detecting B7-H3 or a cell comprising B7-H3, comprising the B7-H3 binding protein of any one of claims 1-12, or the conjugate of any one of claims 17-19.

26. Use of the B7-H3 binding protein of any one of claims 1-12 or the conjugate of any one of claims 17-19 in the manufacture of a kit for detecting B7-H3 or a cell comprising B7-H3.

27. A chimeric antigen receptor comprising an antigen recognition domain, a hinge region, a transmembrane domain, and an intracellular domain comprising a costimulatory domain and a signaling domain, wherein the antigen recognition domain comprises the B7-H3 binding protein of any one of claims 1-12.
